# EUROPEAN PATENT SPECIFICATION

(11) **EP 4 058 028 B1**
(45) Date of publication and mention of the grant of the patent: **05.03.2025**
(21) Application number: 20820686.2
(22) Date of filing: 13.11.2020
(51) Int. Cl.: A61K 31/5377, A61K 9/20, A61K 47/26, A61K 47/36, A61K 31/53, A61P 35/00, A61P 35/02, A61P 43/00

(54) **PEDIATRIC FORMULATIONS FOR TREATMENT OF CANCER**
PÄDIATRISCHE FORMULIERUNGEN ZUR BEHANDLUNG VON KREBS
FORMULATIONS PÉDIATRIQUES POUR LE TRAITEMENT DU CANCER

(30) Priority: 14.11.2019 US 201962935581 P
(43) Date of publication of application: 21.09.2022
(73) Proprietor: Celgene Corporation, Princeton, NJ 08543 (US)
(72) Inventor: MITRA, Biplob K., Summit, NJ 07901 (US); AYCINENA, J. Alex, San Francisco, CA 94158 (US); RUMONDOR, Alfred C.F., Summit, NJ 07901 (US)
(74) Representative: Carpmaels & Ransford LLP
(86) International application number: PCT/US2020/060340
(87) International publication number: WO 2021/097160

(56) References cited:
- WO-A1-2019/241504
- US-A1- 2018 064 715
- US-A1- 2018 125 850
- BIYYALA VARSHA ET AL: "Novel solid oral dosage form in pediatric populations - Emphasis on minitablets", INTERNATIONAL JOURNAL OF PHARMACEUTICS, vol. 511, no. 2, 25 September 2016 (2016-09-25), pages 1145, XP029716295, ISSN: 0378-5173, DOI: 10.1016/J.IJPHARM.2016.06.101
- HARRIS DAVID ET AL: "Age-appropriate solid oral formulations for pediatric applications with a focus on multiparticulates and minitablets: Summary of September 2019 EuPFI workshop", EUROPEAN JOURNAL OF PHARMACEUTICS AND BIOPHARMACEUTICS, ELSEVIER SCIENCE PUBLISHERS B.V., AMSTERDAM, NL, vol. 153, 21 June 2020 (2020-06-21), pages 222 - 225, XP086209566, ISSN: 0939-6411, [retrieved on 20200621], DOI: 10.1016/J.EJPB.2020.06.012
- H. LOU ET AL: "Development of a Mini-Tablet of Co-Grinded Prednisone-Neusilin Complex for Pediatric Use", AAPS PHARMSCITECH, vol. 14, no. 3, 13 June 2013 (2013-06-13), pages 950 - 958, XP055321701, DOI: 10.1208/s12249-013-9981-x
- BIYYALA VARSHA ET AL: "Novel solid oral dosage form in pediatric populations - Emphasis on minitablets", INTERNATIONAL JOURNAL OF PHARMACEUTICS, vol. 511, no. 2, 1 September 2016 (2016-09-01), NL, pages 1145, XP055776333, ISSN: 0378-5173, DOI: 10.1016/j.ijpharm.2016.06.101

## Description

### RELATED APPLICATIONS

This application claims the benefit of U.S. Provisional Application No. 62/935,581, filed November 14, 2019.

### FIELD

Provided herein are pediatric formulations comprising 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol or a pharmaceutically acceptable salt or solid form thereof, and methods for preparing and using the formulations. In certain embodiments, the pediatric formulations provided herein are used for treating a proliferative disease, such as cancer, characterized by the presence of a mutant allele of IDH2. In certain embodiments, provided herein are the pediatric formulations provided herein for use in methods of treating a proliferative disease, such as cancer, characterized by the presence of a mutant allele of IDH2.

### BACKGROUND

It has been reported that 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol is effective in treating proliferative diseases, including cancers. *See* US Patent Nos. 9,732,062; 9,738,625; 9,694,013; 10,201,543; 10,188,656 and 10,137,130; and US Publication No. US 2018/0311249-A1. This drug is currently marketed in the U.S. by Celgene Corporation, as once-daily oral tablets for the treatment of adult patients with relapsed or refractory acute myeloid leukemia (AML) who have an IDH2 mutation, under the trade name IDHIFA^{®}. US Publication No. US 2018/064715-A1 discloses a tablet comprising, as an active ingredient, 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol or a pharmaceutically acceptable salt thereof, wherein the compound is present in an amount from about 15% to about 40% by weight based on total weight of the tablet, an intragranular excipient and an extragranular excipient, wherein the extragranular excipient comprises from about 5% to about 50% microcrystalline cellulose by weight based on total weight of the tablet.

There is a need to develop pediatric formulations comprising 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol or a pharmaceutically acceptable salt or solid form thereof that have good manufacturability, dissolution, stability and bioavailability.

### SUMMARY

In certain embodiments, provided herein are pharmaceutical compositions comprising minitablets, wherein each minitablet comprises 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate and one or more excipients selected from a binder, a sweet diluent, a disintegrant, a wetting agent, a flow agent, a stabilizer, a high intensity sweetener and a lubricant.

Also provided herein are methods of preparing the pharmaceutical compositions provided herein.

Further provided herein are methods of treating and managing various diseases or disorders comprising administering to a pediatric patient a therapeutically effective amount of a pharmaceutical composition provided herein. Any references to methods of treatment by therapy or surgery or in vivo diagnosis methods of this description is to be interpreted as a reference to compounds, pharmaceutical compositions and medicaments of the present invention for use in those methods.

In certain embodiments, provided herein are methods of treating hematologic malignancies or solid tumors, each characterized by the presence of a mutant allele of IDH2 comprising administering a pharmaceutical composition provided herein.

In one embodiment, the hematologic malignancy is selected from acute myelogenous leukemia (AML), myelodysplastic syndrome (MDS), chronic myelomonocytic leukemia (CMML), myeloid sarcoma, multiple myeloma, lymphoma (*e.g*., T-cell lymphoma or B-cell lymphoma), angioimmunoblastic T-cell lymphoma (AITL), blastic plasmacytoid dendritic cell neoplasm and myeloproliferative neoplasm (MPN), each characterized by the presence of a mutant allele of IDH2.

In one embodiment, the solid tumor is selected from glioma, melanoma, chondrosarcoma, and cholangiocarcinoma, each characterized by the presence of a mutant allele of IDH2.

In certain embodiments, provided herein is a pharmaceutical composition for use in the methods of treating and managing diseases or disorders provided herein.

In certain embodiments, the pharmaceutical composition provided herein is used for oral administration in pediatric patients for treating a proliferative disease, such as cancer, characterized by the presence of a mutant allele of IDH2.

### BRIEF DESCRIPTION OF DRAWINGS

Figure 1 is an X-ray powder diffractogram (XRPD) of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate Form 3.
Figure 2 is a differential scanning calorimetry (DSC) profile of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate Form 3.
Figure 3 is a thermal gravimetric analysis (TGA) profile of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate Form 3.
Figure 4 is a dynamic vapor sorption (DVS) profile of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate Form 3.
Figure 5 provides results of a palatability study of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol powder neat and granules prepared according to the adult formulation in IDHIFA^{®}.
Figure 6a illustrates sticking issues for tooling when minitablets were prepared according to the adult formulation in IDHIFA^{®}.
Figure 6b illustrates no sticking or filming issues for tooling when minitablets were prepared according to formulation A-11.
Figure 7 provides results for a palatability assessment for the minitablet formulation described in Table 10.
Figure 8 illustrates a particle size distribution of milled granules for formulation A-12. In the figure, F1, F2, and F3 refer to granules obtained at roll pressures 4, 2, and 0 kN/cm, respectively.
Figure 9 provides a plot of shear cell flow property measurement for formulation A-13 before and after roller compaction unit operation.
Figure 10 provides a plot of tensile strength vs. solid fraction of minitablets having formulation A-13.
Figure 11 illustrates stratified content uniformity from different formulation batches.
Figure 12 provides comparison of multi-media dissolution of minitablets vs. adult tablets IDHIFA^{®}.
Figure 13 provides dissolution profiles for tablets containing 0 and 25% amorphous 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol in multi pH media (top) and biorelevant media FeSSIF (fed state simulating intestinal fluid) (bottom).
Figure 14 provides a process flow diagram for a manufacturing process for an exemplary minitablet provided herein.
Figure 15 illustrates a recommended packaging scheme and product presentation for minitablets-in-capsules.
Figure 16 provides a particle size distribution and sieve cut assay analysis for an exemplary minitablet formulation.

### DETAILED DESCRIPTION

The details of construction and the arrangement of components set forth in the following description or illustrated in the drawings are intended to describe non-limiting embodiments. Other embodiments and different ways to practice the invention are expressly included. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

As used herein, the terms "comprising" and "including" can be used interchangeably. The terms "comprising" and "including" are to be interpreted as specifying the presence of the stated features or components as referred to, but does not preclude the presence or addition of one or more features, or components, or groups thereof. Additionally, the terms "comprising" and "including" are intended to include examples encompassed by the term "consisting of". Consequently, the term "consisting of" can be used in place of the terms "comprising" and "including" to provide for more specific embodiments of the invention.

The term "consisting of" means that a subject-matter has at least 90%, 95%, 97%, 98% or 99% of the stated features or components of which it consists. In another embodiment the term "consisting of" excludes from the scope of any succeeding recitation any other features or components, excepting those that are not essential to the technical effect to be achieved.

As used herein, the term "or" is to be interpreted as an inclusive "or" meaning any one or any combination. Therefore, "A, B or C" means any of the following: "A; B; C; A and B; A and C; B and C; A, B and C". An exception to this definition will occur only when a combination of elements, functions, steps or acts are in some way inherently mutually exclusive.

### 1. Definitions

As used above, and throughout the description of the invention, the following terms, unless otherwise indicated, shall be understood to have the following meanings.

As used in this application, the singular form "a", "an" and "the" include plural references unless the context clearly dictates otherwise. For example, the term "an intragranular excipient" includes one or more intragranular excipients.

As used herein, the term "minitablet" refers to compressed tablets with diameter and height between one to four millimeters (mm).

"Compound 1" is meant to describe 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol or methanesulfonate salt thereof, including solid forms of the free base and methanesulfonate salt.

"Compound 1A" as used herein refers to 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate, also known as enasidenib, including solid forms thereof.

"Compound 1B" as used herein refers to solid form 3 of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate.

"Compound 1C" is meant to describe 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol, including solid forms thereof.

"Compound 1D" is meant to describe amorphous form of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol.

The terms "AG-221" or "AG221" refer to 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol, including solid forms thereof, or 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate, including solid forms thereof.

The term "solid form" refers a crystal form or an amorphous form or a mixture thereof of 2-methyl-1-[(4-[6-(trifluoromethyl)pyridin-2-yl]-6-{ [2-(trifluoromethyl)pyridin-4-yl]amino}-1,3,5-triazin-2-yl)amino]propan-2-ol or 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate. Certain solid forms of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol and its methanesulfonate salt are described in US Patent Nos. 9,738,625 and 10,201,543.

As used herein, and unless otherwise specified, a crystalline or amorphous form that is "pure," *i.e.,* substantially free of other crystalline or amorphous forms, contains less than about 10% by weight of one or more other crystalline or amorphous forms, less than about 5% by weight of one or more other crystalline or amorphous forms, less than about 3% by weight of one or more other crystalline or amorphous forms, or less than about 1% by weight of one or more other crystalline or amorphous forms.

As used herein, API refers to "active pharmaceutical ingredient". In one embodiment, the API is Compound 1. In one embodiment, the API is Compound 1A. In one embodiment, the API is Compound 1B. In one embodiment, the API is Compound 1C. The API may be present in an amorphous state or crystalline form.

As used herein, the terms "inhibit" or "prevent" include both complete and partial inhibition and prevention. An inhibitor may completely or partially inhibit the intended target.

The term "treat" means decrease, suppress, attenuate, diminish, arrest, or stabilize the development or progression of a disease/disorder (i.e., a disease such as AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma, lessen the severity of the disease/disorder (i.e., a disease selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma), each characterized by the presence of a mutant allele of IDH2, or improve the symptoms associated with the disease/disorder (i.e., a disease such as AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, or cholangiocarcinoma), each characterized by the presence of a mutant allele of IDH2.

As used herein, and unless otherwise specified, the terms "prevent," "preventing" and "prevention" refer to the prevention of the onset, recurrence or spread of a disease or disorder, or of one or more symptoms thereof. The terms "prevent," "preventing" and "prevention" contemplate an action that occurs before a patient begins to suffer from the specified disease or disorder or symptoms thereof, which inhibits or reduces the severity of the disease or disorder.

As used herein, and unless otherwise indicated, the terms "manage," "managing" and "management" encompass preventing the recurrence of the specified disease or disorder in a patient who has already suffered from the disease or disorder, or lengthening the time that a patient who has suffered from the disease or disorder remains in remission. The terms encompass modulating the threshold, development or duration of the disease or disorder, or changing the way that a patient responds to the disease or disorder.

The treatment of a cancer may be assessed by Response Evaluation Criteria in Solid Tumors (RECIST 1.1) (*see* Thereasse P., et al. J. of the National Cancer Institute; 2000; (92) 205-216 and Eisenhauer et al. European J. Cancer; 2009; (45) 228-247). Overall responses for all possible combinations of tumor responses in target and non-target lesions with or without the appearance of new lesions are as follows:

| Target lesions | Non-target lesions | New lesions | Overall response | Best response for this category also requires |
|---|---|---|---|---|
| CR | CR | No | CR | Two objective status determinations of CR (not less than 4 weeks apart) before progression |
| CR | Non-CR/Non-PD | No | PR | |
| CR | Not evaluated | No | PR | |
| PR | Non-PD or not all evaluated | No | PR | Two determinations of PR or better (not less than 4 weeks apart) before progression, but not qualifying for CR |
| Stable disease | Non-PD or not all evaluated | No | Stable disease | Documented at least >6 weeks from baseline |
| Not all evaluated | Non-PD | No | NE | |
| PD | Any | Yes or No | PD | |
| Any | PD | Yes or No | PD | |
| Any | Any | Yes | PD | |

| | | | | |
|---|---|---|---|---|
| CR=complete response, NE=not evaluable, PR=partial response, PD=progressive disease | | | | |

The subjects with a global deterioration of health status requiring discontinuation of treatment without objective evidence of disease progression at that time are referred as "symptomatic deterioration".

Response for subjects with non-target lesions only are expressed as follows:

| Non-target lesions | New lesions | Overall response |
|---|---|---|
| CR | No | CR |
| Non-CR/non-PD | No | Non-CR/Non-PD^{a} |
| Not evaluated | No | NE |
| Unequivocal PD | Yes or No | PD |
| Any | Yes | PD |

| | | |
|---|---|---|
| CR=complete response, NE = not evaluable, PD=progressive disease ^{a}Non-CR/non-PD is preferred over "stable disease" for non-target disease. | | |

For assessment of high-grade glioma (HGG), the RANO high-grade glioma criteria as outlined below are used; however, tumor size is determined by the product of the maximal cross-sectional fluid attenuated inversion recovery (FLAIR) diameters instead of enhancing diameters.

### Summary of RANO Response Criteria

| Summary of the Proposed RANO Response Criteria | | | | |
|---|---|---|---|---|
| Criterion | CR | PR | SD | PD |
| T1 gadolinium enhancing disease | None | ≥50% ↓ | <50% ↓ but <25% ↑ | ≥25% ↑* |
| T2/FLAIR | Stable or ↓ | Stable or ↓ | Stable or ↓ | ↑* |
| New lesion | None | None | None | Present* |
| Corticosteroids | None | Stable or ↓ | Stable or ↓ | NA^{†} |
| Clinical status | Stable or ↑ | Stable or ↑ | Stable or ↑ | ↓* |
| Requirement for response | All | All | All | Any* |

| | | | | |
|---|---|---|---|---|
| RANO: Response Assessment in Neuro-Oncology; CR: complete response; PR: partial response; SD: stable disease; PD: progressive disease; FLAIR: fluid-attenuated inversion recovery; NA: nonapplicable. *Progression occurs when this criterion is present. †Increase in corticosteroids alone will not be taken into account in determining progression in the absence of persistent clinical deterioration. | | | | |

Summary of International neuroblastoma response criteria (INRC) working group metastatic response criteria (soft tissue/ bone) is provided below:

| Response | Anatomical imaging + MIBG (FDG-PET) |
|---|---|
| CR | 1. Complete resolution of non-primary measurable lesions |
| | 2. MIBG non-avid (no increased FDG-PET, uptake for MIBG non-avid tumors) of non-primary lesions |
| PR | 1. ≥30% decrease in size/sum of non-primary measurable disease (RECIST), OR |
| | 2. ≥50% reduction in MIBG score (relative MIBG score >0 to ≤to 0.5) |
| MR | 1. CR or PR for one compartment (bone or soft-tissue) with at least SD in the other as long as no PD |
| PD | 1. Any new lesion by CT/MRI or MIBG |
| | 2. ≥20% increase in size AND a minimum absolute increase of 5mm in longest dimension in existing lesions |
| | 3. Relative MIBG (FDG-PET for MIBG non-avid tumors) score ≥1.2 |
| SD | 1. Neither sufficient shrinkage for MRI or PR nor sufficient increase for PD |

| | |
|---|---|
| CR: complete response; PR: partial response; MR: minor response; PD: progressive disease SD: stable disease | |

As used herein, "measurable lesions" are defined as lesions that can be accurately measured in at least one dimension (longest diameter to be recorded) with a minimum size of:
10 mm by CT scan (CT scan slice thickness no greater than 5 mm) or MRI. If scans with slice thicknesses greater than 5 mm are used, the minimum size should be twice the slice thickness.
20 mm by chest x-ray.
10 mm caliper measurement by clinical examination (lesions which cannot be accurately measured with calipers should be recorded as non-measurable)

As used herein, the term "target lesions" refers to all measurable lesions up to a maximum of 2 lesions per organ and 5 lesions in total, representative of all involved organs are identified as target lesions and be recorded and measured at baseline. These 5 lesions are selected on the basis of their size (lesions with the longest diameter), be representative of all involved organs and are suitable for reproducible repeated measurements. A sum of the diameters (longest for non-nodal lesions, short axis for nodal lesions) for all target lesions is calculated and reported as the baseline sum diameters. The baseline sum diameters are used as reference to further characterize any objective tumor regression of the measurable dimension of the disease. If there are > 5 measurable lesions, those not selected as target lesions will be considered together with non-measurable disease as non-target lesions.

As used herein, the term "non-target lesions" refers to all non-measurable lesions (or sites of disease) plus any measurable lesions over and above the 5 listed as target lesions. These lesions are noted at baseline and followed as "present", "absent" or in rare cases "unequivocal progression".

As used herein, the term "complete response" or "CR" refers to disappearance of all clinical and radiological evidence of tumor (both target and non-target). Any pathological lymph nodes (whether target or non target) must have a reduction in short axis to <10 mm.

As used herein, the term "partial response" or "PR" refers to at least a 30% decrease in the sum of diameters of target lesions taking as reference the baseline sum, no unequivocal progression of existing non target lesions and no appearance of new lesions.

As used herein, the term "stable disease" or "SD" refers to steady state of disease. Neither sufficient shrinkage to qualify for PR nor sufficient increase to qualify for progressive disease (PD), no unequivocal progression of existing non-target lesions and no appearance of new lesions.

As used herein, the term "progressive disease" or "PD" refers to at least a 20% increase in the sum of diameters of target lesions taking as reference the smallest sum on study (this includes the baseline sum if that is the smallest on study). In addition to the relative increase of 20%, the sum must also demonstrate an absolute increase of at least 5 mm. Unequivocal progression of existing non target lesions or the appearance of one or more new lesions also constitutes progressive disease.

As used herein, the term "overall response rate" or "ORR" means the sum of the percentage of patients who achieve complete and partial responses. Duration of response (DOR) is the time from achieving a response until relapse or disease progression.

As used herein, the term, overall survival' or "OS" means the time from randomization in a clinical trial until death from any cause. Progression-free survival (PFS) means the time from randomization in a clinical trial until progression or death. Event-free survival (EFS) means the time from study entry until any treatment failure, including disease progression, treatment discontinuation for any reason, or death.

The treatment of cancer in pediatric patients may be assessed based on Karnofsky performance status (for patients >12 years of age) or Lansky Play score (for patients ≤12 years of age). ≥70%. Patients who are unable to walk because of paralysis or stable neurological disability, but who are up in a wheelchair, will be considered ambulatory for the purpose of assessing the performance score.

| Lansky performance score (Patients ≤ 12 years old) | |
|---|---|
| 100 | Fully active, normal |
| 90 | Minor restrictions in physically strenuous activity |
| 80 | Active, but tires more quickly |
| 70 | Both greater restriction of play and less time spent in play activity |
| 60 | Up and around, but minimal active play; keeps busy with quieter activities |
| 50 | Gets dressed but lies around much of the day; no active play but able to participate in all quiet play and activities |
| 40 | Mainly in bed; participates in quiet activities |
| 30 | Bed-bound; needs assistance even for quiet play |
| 20 | Often sleeping; play entirely limited to very passive activities |
| 10 | No play; does not get out of bed |
| 0 | Unresponsive |

| Karnofsky performance score (Patients > 12 years old) | | |
|---|---|---|
| Able to carry on normal activity and work; no special care needed | 100 | Normal; no complaints |
| | 90 | Able to carry on normal activity; minor signs or symptoms of disease |
| | 80 | Normal activity with effort; some signs or symptoms of disease |
| Able to carry on normal activity and work; no special care needed | 70 | Cares for self; unable to carry on normal activity or work |
| | 60 | Requires occasional assistance; able to care for most personal needs |
| | 50 | Requires considerable assistance and frequent medical care |
| Unable to care for self; requires equivalent of institutional or hospital care; disease may be progressing rapidly | 40 | Disabled; requires special care and assistance |
| | 30 | Severely disabled; hospitalization is indicated though death not imminent |
| | 20 | Very sick; hospitalization necessary; active supportive treatment necessary |
| | 10 | Moribund; fatal processes progressing rapidly |
| | 0 | Dead |

As used herein, and unless otherwise specified, a "therapeutically effective amount" of a compound is an amount sufficient to provide a therapeutic benefit in the treatment or management of a disease or disorder, or to delay or minimize one or more symptoms associated with the disease or disorder. A therapeutically effective amount of a compound means an amount of therapeutic agent, alone or in combination with other therapies, that provides a therapeutic benefit in the treatment or management of the disease or disorder. The term "therapeutically effective amount" can encompass an amount that improves overall therapy, reduces or avoids symptoms or causes of disease or disorder, or enhances the therapeutic efficacy of another therapeutic agent.

As used herein, and unless otherwise specified, a "prophylactically effective amount" of a compound is an amount sufficient to prevent a disease or disorder, or one or more symptoms thereof, or prevent the recurrence of the disease or disorder, or one or more symptoms thereof. A prophylactically effective amount of a compound means an amount of therapeutic agent, alone or in combination with other agents, that provides a prophylactic benefit in the prevention of the disease or disorder. The term "prophylactically effective amount" can encompass an amount that improves overall prophylaxis or enhances the prophylactic efficacy of another prophylactic agent.

Unless otherwise specified, the term "composition" as used herein is intended to encompass a product comprising the specified ingredient(s) (and in the specified amount(s), if indicated), as well as any product which results, directly or indirectly, from combination of the specified ingredient(s) in the specified amount(s).

A "pharmaceutically acceptable excipient, diluent or carrier," refers to a substance that aids the administration of an active agent to a subject by for example by modifying the stability of an active agent or modifying the absorption by a subject upon administration. A pharmaceutically acceptable excipient typically has no significant adverse toxicological effect on the patient. Examples of pharmaceutically acceptable excipients include, for example bulking agents, buffers, binders, fillers, disintegrants, lubricants, coatings, sweeteners, flavors, fatty acid esters, hydroxymethycellulose, polyvinyl pyrrolidine, and colors, and the like. One of skill in the art will recognize that other pharmaceutical excipients known in the art are useful in the present invention and include those listed in for example the Handbook of Pharmaceutical Excipients, Rowe R.C., Shesky P.J., and Quinn M.E., 6th Ed., The Pharmaceutical Press, RPS Publishing (2009). The terms "bulking agent", and "buffer" are used in accordance with the plain and ordinary meaning within the art.

As used herein, "administer" or "administration" refers to the act of physically delivering a substance as it exists outside the body into a subject. Administration includes all forms known in the art for delivering therapeutic agents, including but not limited to oral, topical, mucosal, injections, intradermal, intravenous, intramuscular delivery or other method of physical delivery described herein or known in the art (e.g., implantation of a slow-release device, such as a mini-osmotic pump to a subject; liposomal formulations; buccal; sublingual; palatal; gingival; nasal; vaginal; rectal; intra-arteriole; intraperitoneal; intraventricular; intracranial; or transdermal).

The term "co-administer" as used herein with respect to an additional cancer therapeutic agents means that the additional cancer therapeutic agent may be administered prior to, consecutively with, or following the administration of a composition provided herein. In such combination therapy treatment, the second therapeutic agent(s) is administered by conventional methods.

As used herein, the term "pediatric patient" refers to a patient 21 years or younger, in certain embodiments, a patient 18 years or younger, in certain embodiments, a patient 16 years or younger, in certain embodiments, a patient 14 years or younger, in certain embodiments, a patient 12 years or younger, in certain embodiments, a patient 10 years or younger, or in certain embodiments, a patient 8 years or younger, or in certain embodiments, a patient 6 years or younger.

As used herein, and unless otherwise specified, the terms "about" and "approximately," when used in connection with doses, amounts, or weight percents of ingredients of a composition or a dosage form, mean a dose, amount, or weight percent that is recognized by one of ordinary skill in the art to provide a pharmacological effect equivalent to that obtained from the specified dose, amount, or weight percent. In certain embodiments, the terms "about" and "approximately," when used in this context, contemplate a dose, amount, or weight percent within 30%, within 20%, within 15%, within 10%, or within 5%, of the specified dose, amount, or weight percent.

Unless otherwise specified, to the extent that there is a discrepancy between a depicted chemical structure of a compound provided herein and a chemical name of a compound provided herein, the chemical structure shall control.

### 2. Compound

In certain embodiments, provided herein are pediatric formulations comprising 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate (Compound 1A). 2-Methyl-1-[(4-[6-(trifluoromethyl)pyridin-2-yl]-6-{[2-(trifluoromethyl)pyridin-4-yl]amino}-1,3,5-triazin-2-yl)amino]propan-2-ol has the following formula:

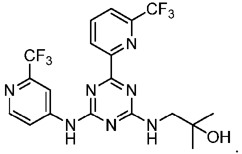

In one embodiment, Compound 1A used in the pediatric formulations provided herein is a crystalline solid. In one embodiment, the pediatric formulations provided herein comprise a solid form of Compound 1A. In one embodiment, the pediatric formulations provided herein comprise Compound 1B.

In one embodiment, Compound 1, Compound 1B, Compound 1C and Compound 1D can be synthesized and used according to the methods described in U.S. Patent Nos. 9,512,107; 9,656,999; 9,732,062; 9,738,625; 9,751,863 and U.S. Publication No. 2017/0305885 A1, and PCT Publication No. WO 2016/126798.

The solid forms of Compound 1A, including Compound 1B, are described in US Patent Nos. 9,738,625 and 10,201,543.

### 3. Compound 1B

In one embodiment, the minitablets provided herein comprise Compound 1B.

In one embodiment, Compound 1B is prepared by contacting a solution of Compound 1C in acetone with methanesulfonic acid (MSA)/acetone solution.

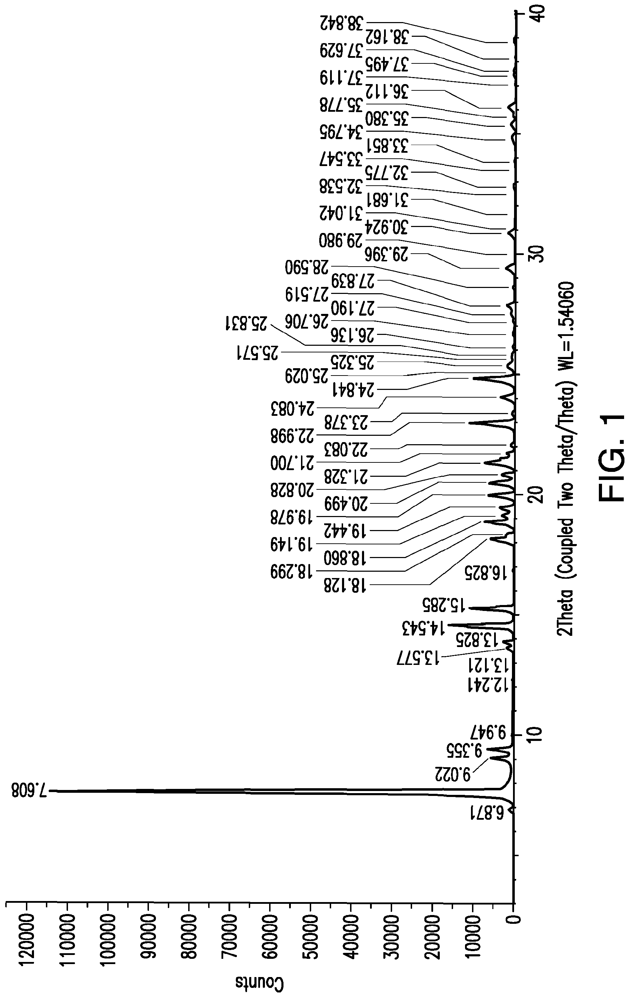

In certain embodiments, Compound 1B is characterized by the X-ray powder diffraction (XRPD) pattern shown in Figure 1, and data shown in Table A, obtained using CuKa radiation. In a particular embodiment, the solid form is characterized by one or more of the peaks taken from Figure 1, as shown in Table A. For example, the solid form is characterized by one or two or three or four or five or six or seven or eight or nine or ten of the peaks shown in Table A.

**Table A**

| **Angle 2-Theta°** | **Intensity %** |
|---|---|
| 7.5 | 100.0 |
| 9.0 | 16.5 |
| 9.3 | 27.2 |
| 14.5 | 48.5 |
| 15.2 | 17.2 |
| 18.0 | 17.0 |
| 18.8 | 32.6 |
| 19.9 | 18.7 |
| 21.3 | 19.3 |
| 24.8 | 33.8 |

In another embodiment, Compound 1B is characterized by the peaks identified at 2θ angles of 7.5, 9.3, 14.5, 18.8, 21.3, and 24.8°. In a further embodiment, Compound 1B is characterized by the peaks are identified at 2θ angles of 7.5, 14.5, 18.8, and 24.8°. In another, embodiment, Compound 1B is characterized by the peaks identified at 2θ angles of 7.5, 14.5, and 24.8°.

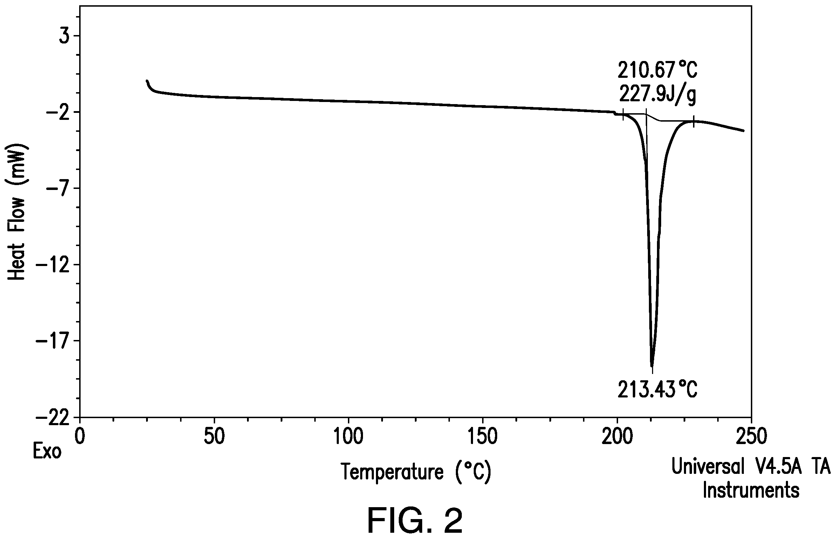

In another embodiment, Compound 1B is characterized by the differential scanning calorimetry profile (DSC) shown in Figure 2. The DSC graph plots the heat flow as a function of temperature from a sample, the temperature rate change being about 10 °C /min. The profile is characterized by a strong endothermic transition with an onset temperature of about 210.7 °C with a melt at about 213.4 °C.

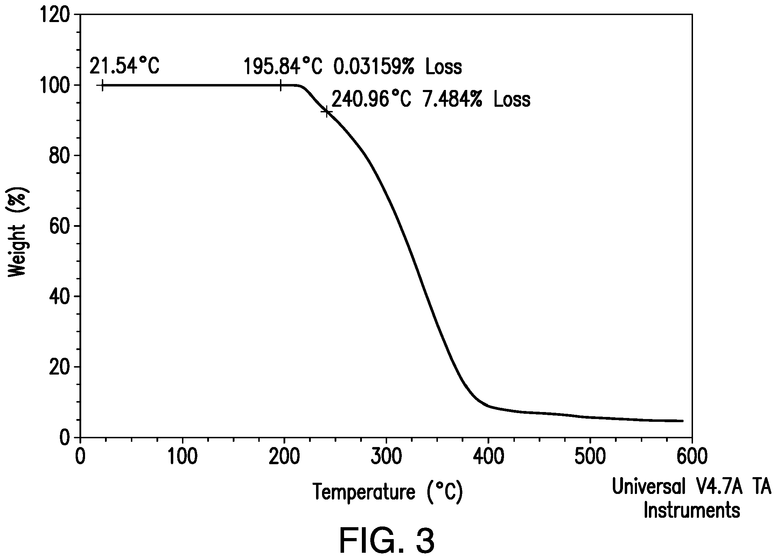

In another embodiment, Compound 1B is characterized by thermal gravimetric analysis (TGA) shown in Figure 3. The TGA profile graphs the percent loss of weight of the sample as a function of temperature, the temperature rate change being about 10 °C /min. The weight loss represents a loss of about 0.03% of the weight of the sample as the temperature is changed from about 21°C to 196 °C and about 7.5% of the weight of the sample as the temperature is changed from about 196°C to 241°C.

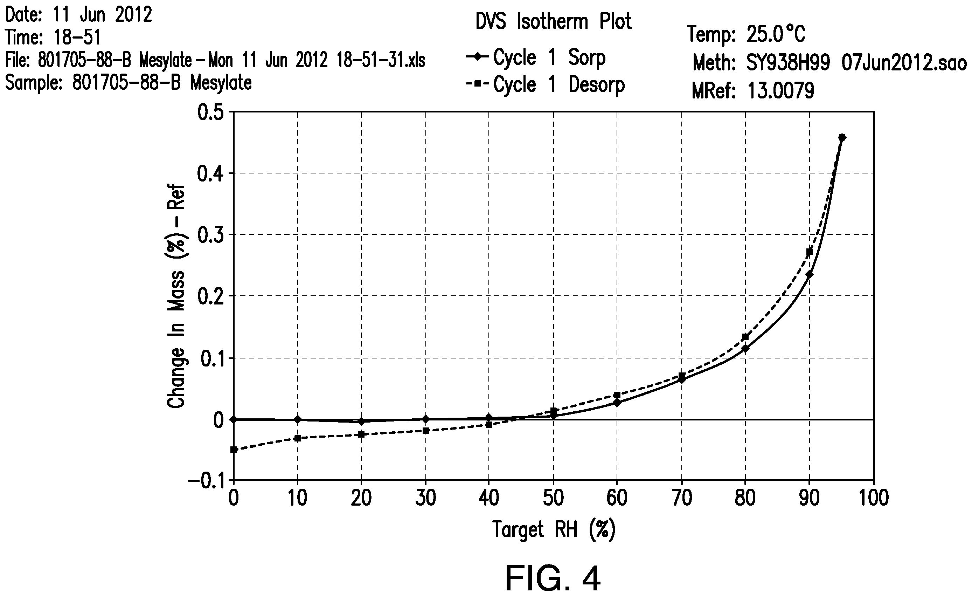

In another embodiment, Compound 1B is characterized by an X-ray powder diffraction pattern substantially similar to Figure 1. In another embodiment, Compound 1B is characterized by a differential scanning calorimetry (DSC) profile substantially similar to Figure 2. In another embodiment, Compound 1B is characterized by a thermal gravimetric analysis (TGA) profile substantially similar to Figure 3. In further embodiments, Compound 1B is characterized by one or more of the features listed in this paragraph. In another embodiment, Compound 1B is characterized by a DYS profile substantially similar to Figure 4.

### 4. Formulations

In certain embodiments, the pediatric formulations provided herein are minitablets comprising Compound 1A and a pharmaceutically acceptable excipient. In certain embodiments, the pediatric formulations provided herein are minitablets comprising Compound 1B and a pharmaceutically acceptable excipient. In certain embodiments, the minitablet provided herein comprises Compound 1A in about 7% to about 12% by weight based on total weight of the minitablet. In certain embodiments, the minitablet provided herein comprises Compound 1B in about 7% to about 12% by weight based on total weight of the minitablet. In certain embodiments, the minitablet provided herein comprises Compound 1A in about 9% to about 10% by weight based on total weight of the minitablet. In certain embodiments, the minitablet provided herein comprises Compound 1B in about 9% to about 10% by weight based on total weight of the minitablet. In certain embodiments, the minitablet composition provided herein comprises Compound 1A in about 7.00%, about 8.00%, about 9.00%, about 9.50%, about 9.60%, about 9.62%, about 9.75%, about 10.00%, about 11.00% or about 12.00% by weight based on total weight of the minitablet. In certain embodiments, the minitablet composition provided herein comprises Compound 1B in about 7.00%, about 8.00%, about 9.00%, about 9.50%, about 9.60%, about 9.62%, about 9.75%, about 10.00%, about 11.00% or about 12.00% by weight based on total weight of the minitablet. In certain embodiments, the minitablet provided herein comprises Compound 1A in an amount of about 9.6% by weight based on total weight of the minitablet. In certain embodiments, the minitablet provided herein comprises Compound 1B in an amount of about 9.6% by weight based on total weight of the minitablet. In certain embodiments, the minitablet provided herein comprises Compound 1 in an amount of about 9.62% by weight based on total weight of the minitablet. In certain embodiments, the minitablet provided herein comprises Compound 1B in an amount of about 9.62% by weight based on total weight of the minitablet.

In certain embodiments, the minitablet provided herein comprises Compound 1A and one or more excipients selected from a binder, a sweet diluent, a disintegrant, a wetting agent, a flow agent, a stabilizer, a high intensity sweetener and a lubricant. In certain embodiments, the minitablet provided herein comprises Compound 1B and one or more excipients selected from a binder, a sweet diluent, a disintegrant, a wetting agent, a flow agent, a stabilizer, a high intensity sweetener and a lubricant.

In certain embodiments, the binder is microcrystalline cellulose. In certain embodiments, the binder is present in an amount from about 40% to 50% by weight based on total weight of the minitablet. In certain embodiments, the binder is present in an amount from about 42% to 45% by weight based on total weight of the minitablet. In certain embodiments, the binder is present in an amount of about 42.0%, 43.0%, 43.2%, 44.0%, or 45.0% by weight based on total weight of the minitablet. In certain embodiments, the binder is present in an amount of about 43.2% by weight based on total weight of the minitablet. In certain embodiments, the binder is present in an amount of about 43.24% by weight based on total weight of the minitablet. In certain embodiments, the binder is microcrystalline cellulose, which is present in an amount of about 43.2% by weight based on total weight of the minitablet. In certain embodiments, the binder is microcrystalline cellulose, which is present in an amount of about 43.24% by weight based on total weight of the minitablet.

In certain embodiments, the sweet diluent is mannitol, sucrose or dextrose. In certain embodiments, the sweet diluent is mannitol. In certain embodiments, the sweet diluent is present in an amount from about 25% to 30% by weight based on total weight of the minitablet. In certain embodiments, the sweet diluent is present in an amount from about 26.5% to 28.5% by weight based on total weight of the minitablet. In certain embodiments, the sweet diluent is present in an amount of about 26.5%, 27.0%, 27.2%, 28.0%, or 28.5% by weight based on total weight of the minitablet. In certain embodiments, the sweet diluent is present in an amount of about 27.2% by weight based on total weight of the minitablet. In certain embodiments, the sweet diluent is present in an amount of about 27.24% by weight based on total weight of the minitablet. In certain embodiments, the sweet diluent is mannitol, which is present in an amount of about 27.2% by weight based on total weight of the minitablet. In certain embodiments, the sweet diluent is mannitol, which is present in an amount of about 27.24% by weight based on total weight of the minitablet.

In certain embodiments, the binder is hydroxypropyl cellulose. In certain embodiments, the binder is present in an amount from about 1% to 3% by weight based on total weight of the minitablet. In certain embodiments, the binder is present in an amount from about 1.5% to 2.5% by weight based on total weight of the minitablet. In certain embodiments, the binder is hydroxypropyl cellulose, which is present in an amount from about 1%, 2% or 3% by weight based on total weight of the minitablet. In certain embodiments, the binder is hydroxypropyl cellulose, which is present in an amount of about 2% by weight based on total weight of the minitablet.

In certain embodiments, the disintegrant is sodium starch glycolate. In certain embodiments, the disintegrant is present in an amount from about 6% to 10% by weight based on total weight of the minitablet. In certain embodiments, the disintegrant is sodium starch glycolate. In certain embodiments, the disintegrant is present in an amount from about 7% to 9% by weight based on total weight of the minitablet. In certain embodiments, the disintegrant is present in an amount of about 6%, 7%, 8%, 9% or 10% by weight based on total weight of the minitablet. In certain embodiments, the disintegrant is sodium starch glycolate, which is present in an amount of about 8% by weight based on total weight of the minitablet.

In certain embodiments, the wetting agent is sodium lauryl sulfate. In certain embodiments, the wetting agent is present in an amount from about 0.5% to 1.5% by weight based on total weight of the minitablet. In certain embodiments, the wetting agent is sodium lauryl sulfate, which is present in an amount of about 0.5%, 1% or 1.5% by weight based on total weight of the minitablet. In certain embodiments, the wetting agent is sodium lauryl sulfate, which is present in an amount of about 1% by weight based on total weight of the minitablet.

In certain embodiments, the flow agent is colloidal silicon dioxide. In certain embodiments, the flow agent is present in an amount from about 1% to 3% by weight based on total weight of the minitablet. In certain embodiments, the flow agent is present in an amount from about 1.5% to 2.5% by weight based on total weight of the minitablet. In certain embodiments, the flow agent is colloidal silicon dioxide, which is present in an amount of about 1%, 2% or 3% by weight based on total weight of the minitablet. In certain embodiments, the flow agent is colloidal silicon dioxide which is present in an amount of about 2% by weight based on total weight of the minitablet.

In certain embodiments, the stabilizer is hypromellose acetate succinate. In certain embodiments, the stabilizer is present in an amount from about 0.5% to 2% by weight based on total weight of the minitablet. In certain embodiments, the stabilizer is present in an amount from about 0.5% to 1.5% by weight based on total weight of the minitablet. In certain embodiments, the stabilizer is hypromellose acetate succinate, which is present in an amount of about 0.5%, 1.0% or 1.5% by weight based on total weight of the minitablet. In certain embodiments, the stabilizer is hypromellose acetate succinate, which is present in an amount of about 1% by weight based on total weight of the minitablet.

In certain embodiments, the high intensity sweetener is sucralose. In certain embodiments, the high intensity sweetener is present in an amount from about 3.5% to 5% by weight based on total weight of the minitablet. In certain embodiments, the high intensity sweetener is present in an amount from about 3.5% to 4.5% by weight based on total weight of the minitablet. In certain embodiments, the high intensity sweetener is sucralose, which is present in an amount of about 3.0%, 4.0%, 4.4% or 5.0% by weight based on total weight of the minitablet. In certain embodiments, the high intensity sweetener is sucralose, which is present in an amount of about 4.4% by weight based on total weight of the minitablet.

In certain embodiments, the lubricant is magnesium stearate. In certain embodiments, the lubricant is present in an amount from about 1% to 3% by weight based on total weight of the minitablet. In certain embodiments, the lubricant is present in an amount from about 1% to 2% by weight based on total weight of the minitablet. In certain embodiments, the lubricant is magnesium stearate, which is present in an amount of about 1.0%, 1.5%, 2.0% or 3.0% by weight based on total weight of the minitablet. In certain embodiments, the lubricant is magnesium stearate, which is present in an amount of about 1.5% by weight based on total weight of the minitablet.

In certain embodiments, the minitablet provided herein comprises Compound 1A, microcrystalline cellulose, mannitol, hydroxypropyl cellulose, sodium starch glycolate, sodium lauryl sulfate, colloidal silicon dioxide, hypromellose acetate succinate, sucralose, and magnesium stearate. In certain embodiments, the minitablet provided herein comprises Compound 1B, microcrystalline cellulose, mannitol, hydroxypropyl cellulose, sodium starch glycolate, sodium lauryl sulfate, colloidal silicon dioxide, hypromellose acetate succinate, sucralose, and magnesium stearate.

In certain embodiments, the minitablet provided herein comprises about 7% to about 12% Compound 1A, about 40% to about 50% microcrystalline cellulose, about 25% to about 30% mannitol, about 1% to about 3% hydroxypropyl cellulose, about 6% to about 10% sodium starch glycolate, about 0.5% to about 1.5% sodium lauryl sulfate, about 1% to about 3% colloidal silicon dioxide, about 0.5% to about 2% hypromellose acetate succinate, about 3.5% to 5% sucralose, and about 1% to about 3% magnesium stearate, where the percentages are by weight based on total weight of the minitablet.

In certain embodiments, the minitablet provided herein comprises about 7% to about 12% Compound 1B, about 40% to about 50% microcrystalline cellulose, about 25% to about 30% mannitol, about 1% to about 3% hydroxypropyl cellulose, about 6% to about 10% sodium starch glycolate, about 0.5% to about 1.5% sodium lauryl sulfate, about 1% to about 3% colloidal silicon dioxide, about 0.5% to about 2% hypromellose acetate succinate, about 3.5% to 5% sucralose, and about 1% to about 3% magnesium stearate, where the percentages are by weight based on total weight of the minitablet.

In certain embodiments, the minitablet provided herein comprises about 9% to about 10% Compound 1A, about 42% to about 45% microcrystalline cellulose, about 26.5% to about 28.5% mannitol, about 1% to about 3% hydroxypropyl cellulose, about 6% to about 10% sodium starch glycolate, about 0.5% to about 1.5% sodium lauryl sulfate, about 1% to about 3% colloidal silicon dioxide, about 0.5% to about 2% hypromellose acetate succinate, about 3.5% to 5% sucralose, and about 1% to about 3% magnesium stearate, where the percentages are based on total weight of the minitablet.

In certain embodiments, the minitablet provided herein comprises about 9% to about 10% Compound 1B, about 42% to about 45% microcrystalline cellulose, about 26.5% to about 28.5% mannitol, about 1% to about 3% hydroxypropyl cellulose, about 6% to about 10% sodium starch glycolate, about 0.5% to about 1.5% sodium lauryl sulfate, about 1% to about 3% colloidal silicon dioxide, about 0.5% to about 2% hypromellose acetate succinate, about 3.5% to 5% sucralose, and about 1% to about 3% magnesium stearate, where the percentages are based on total weight of the minitablet.

In certain embodiments, the minitablet provided herein comprises about 9% to about 10% Compound 1A, about 42% to about 45% microcrystalline cellulose, about 26.5% to about 28.5% mannitol, about 1% to about 2% hydroxypropyl cellulose, about 7% to about 9% sodium starch glycolate, about 0.5% to about 1.5% sodium lauryl sulfate, about 1.5% to about 2.5% colloidal silicon dioxide, about 0.5% to about 1.5% hypromellose acetate succinate, about 3.5% to 4.5% sucralose, and about 1.5% to about 2.5% magnesium stearate, where the percentages are based on total weight of the minitablet.

In certain embodiments, the minitablet provided herein comprises about 9% to about 10% Compound 1B, about 42% to about 45% microcrystalline cellulose, about 26.5% to about 28.5% mannitol, about 1% to about 2% hydroxypropyl cellulose, about 7% to about 9% sodium starch glycolate, about 0.5% to about 1.5% sodium lauryl sulfate, about 1.5% to about 2.5% colloidal silicon dioxide, about 0.5% to about 1.5% hypromellose acetate succinate, about 3.5% to 4.5% sucralose, and about 1.5% to about 2.5% magnesium stearate, where the percentages are based on total weight of the minitablet.

In certain embodiments, the minitablet provided herein comprises about 9.6% Compound 1A, about 43.2% microcrystalline cellulose, about 27.2% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, and about 1.5% magnesium stearate, where the percentages are based on total weight of the minitablet.

In certain embodiments, the minitablet provided herein comprises about 9.6% Compound 1B, about 43.2% microcrystalline cellulose, about 27.2% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, and about 1.5% magnesium stearate, where the percentages are based on total weight of the minitablet.

In certain embodiments, the minitablet provided herein comprises about 9.62% Compound 1A, about 43.24% microcrystalline cellulose, about 27.24% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, and about 1.5% magnesium stearate, where the percentages are based on total weight of the minitablet.

In certain embodiments, the minitablet provided herein comprises about 9.62% Compound 1B, about 43.24% microcrystalline cellulose, about 27.24% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, and about 1.5% magnesium stearate, where the percentages are based on total weight of the minitablet.

In certain embodiments, the pediatric formulation provided herein comprises minitablets encapsulated in a capsule. In certain embodiments, the pediatric formulation provided herein comprises minitablets encapsulated in a hypomellose capsule. In certain embodiments, the hypomellose capsule is size 00 Vcaps Plus Swedish orange hypromellose capsule. In certain embodiments, each capsule comprises about 100-500 minitablets. In certain embodiments, each capsule comprises about 100, 150, 200, 250, 300, 350, 400, 450 or 500 minitablets. In certain embodiments, each capsule comprises about 150 minitablets. In certain embodiments, each capsule comprises about 300 minitablets. In certain embodiments, each capsule comprises about 450 minitablets. The capsule is opened to sprinkle the minitablets onto soft foods, such as yogurt, gelatin snack, and pudding.

In certain embodiments, each minitablet weighs about 1 mg to about 3 mg. In certain embodiments, each minitablet weighs about 1.00 mg, 1.50 mg, 1.67 mg, 2.00 mg or 3.00 mg. In certain embodiments, each minitablet weighs about 1.67 mg. In certain embodiments, each minitablet has a diameter between about 1 mm to about 3 mm. In certain embodiments, each minitablet has a diameter of about 1 mm, 1.2 mm, 1.5 mm, 1.8 mm, 2 mm, 2.4 mm, 2.8 mm or 3 mm. In certain embodiments, each minitablet has a diameter of about 1.2 mm. In certain embodiments, each minitablet has a height between about 1 mm to about 3 mm. In certain embodiments, each minitablet has a height of about 1 mm, 1.2 mm, 1.5 mm, 1.8 mm, 2 mm, 2.4 mm, 2.8 mm or 3 mm. In certain embodiments, each minitablet has a height of 1.2 mm.

In certain embodiments, the minitablet provided herein comprises about 9.62% Compound 1A, about 43.24% microcrystalline cellulose, about 27.24% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, about 1.5% magnesium stearate, where the percentages are based on total weight of the minitablet, the minitablet weighs about 1.67 mg and has a diameter of about 1.2 mm.

In certain embodiments, the minitablet provided herein comprises about 9.62% Compound 1B, about 43.24% microcrystalline cellulose, about 27.24% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, about 1.5% magnesium stearate, where the percentages are based on total weight of the minitablet, the minitablet weighs about 1.67 mg and has a diameter of about 1.2 mm.

In certain embodiments, each capsule comprises about 24.05 mg Compound 1A, about 108.1 mg microcrystalline cellulose, about 68.1 mg mannitol, about 5.0 mg hydroxypropyl cellulose, about 20.0 mg sodium starch glycolate, about 2.5 mg sodium lauryl sulfate, about 5.0 mg colloidal silicone dioxide, about 2.5 mg hypromellose acetate succinate, about 11.0 mg sucralose, and about 3.75 mg magnesium stearate.

In certain embodiments, each capsule comprises about 24.05 mg Compound 1B, about 108.1 mg microcrystalline cellulose, about 68.1 mg mannitol, about 5.0 mg hydroxypropyl cellulose, about 20.0 mg sodium starch glycolate, about 2.5 mg sodium lauryl sulfate, about 5.0 mg colloidal silicone dioxide, about 2.5 mg hypromellose acetate succinate, about 11.0 mg sucralose, and about 3.75 mg magnesium stearate.

In certain embodiments, the minitablets provided herein have a solid fraction of about 0.8 to 0.98. In certain embodiments, the minitablets provided herein have a solid fraction of about 0.9 to 0.96. In certain embodiments, the minitablets provided herein have a tensile strength of 0.5 to 2.0 MPa. In certain embodiments, the minitablets provided herein have a tensile strength of 0.75 to 2.0 MPa. In certain embodiments, the minitablets provided herein have a tensile strength of 0.75 to 1.85 MPa. In certain embodiments, the minitablets provided herein have a solid fraction of about 0.86 to 0.96 and a tensile strength of 0.75 to 1.85 MPa.

In certain embodiments, provided herein is a capsule comprising about 100-500 minitablets comprising Compound 1A. In certain embodiments, provided herein is a capsule comprising about 150, 300 or 450 minitablets comprising Compound 1A. In certain embodiments, provided herein is a capsule comprising about 150 minitablets comprising Compound 1A. In certain embodiments, provided herein is a capsule comprising about 300 minitablets comprising Compound 1A. In certain embodiments, provided herein is a capsule comprising about 450 minitablets comprising Compound 1A. In certain embodiments, provided herein is a capsule comprising about 100-500 minitablets comprising Compound 1B. In certain embodiments, provided herein is a capsule comprising about 150, 300 or 450 minitablets comprising Compound 1B. In certain embodiments, provided herein is a capsule comprising about 150 minitablets comprising Compound 1B. In certain embodiments, provided herein is a capsule comprising about 300 minitablets comprising Compound 1B. In certain embodiments, provided herein is a capsule comprising about 450 minitablets comprising Compound 1B.

In certain embodiments, provided herein is a capsule comprising about 150 minitablets, wherein each minitablet comprises about 9.62% Compound 1A, about 43.24% microcrystalline cellulose, about 27.24% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, and about 1.5% magnesium stearate, where the percentages are based on total weight of the minitablet, the minitablet weighs about 1.67 mg and has a diameter of about 1.2 mm.

In certain embodiments, provided herein is a capsule comprising about 150 minitablets, wherein each minitablet comprises about 9.62% Compound 1B, about 43.24% microcrystalline cellulose, about 27.24% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, and about 1.5% magnesium stearate, where the percentages are based on total weight of the minitablet, the minitablet weighs about 1.67 mg and has a diameter of about 1.2 mm.

In certain embodiments, the capsules are packaged in HDPE bottles with induction-sealed child-resistant closure. In certain embodiments, each bottle comprises a 2-g desiccant canister to provide extra protection against moisture. In certain embodiments, the bottles are packaged in aluminum pouches. In certain embodiments, the aluminum pouches are heat sealed. In certain embodiments, the bottles are stored at refrigerated (2-8 ⁰C) temperature to maximize the shelf life of formulation.

### 5. Methods of Preparation

Any conventional method for obtaining minitablets, and capsules comprising the minitablets can be used, for example, the methods described in pharmacopoeias such as the U.S. Pharmacopeia, and the European Pharmacopoeia, may be used.

In certain embodiments, the method for making a minitablet comprises the steps of mixing, blending, roller compaction, final blending, and compression. In certain embodiments, the method for making a capsule comprising the minitablets comprises the steps of mixing, blending, roller compaction, final blending, compression, and capsule filling. In certain embodiments, provided herein is a method for making a capsule comprising about 100-500 minitablets comprising Compound 1A. In certain embodiments, provided herein is a method for making a capsule comprising about 150, 300 or 450 minitablets comprising Compound 1A. In certain embodiments, provided herein is a method for making a capsule comprising about 150 minitablets comprising Compound 1A. In certain embodiments, provided herein is a method for making a capsule comprising about 100-500 minitablets comprising Compound 1B. In certain embodiments, provided herein is a method for making a capsule comprising about 150, 300 or 450 minitablets comprising Compound 1B. In certain embodiments, provided herein is a method for making a capsule comprising about 150 minitablets comprising Compound 1B.

In certain embodiments, provided herein is a method for making a capsule comprising about 150 minitablets, wherein each minitablet comprises Compound 1B. In certain embodiments, provided herein is a method for making a capsule comprising about 300 minitablets, wherein each minitablet comprises Compound 1B. In certain embodiments, provided herein is a method for making a capsule comprising about 450 minitablets, wherein each minitablet comprises Compound 1B.

In one embodiment, the method for making a minitablet comprises one or more of the following steps: blending intragranular components to obtain an intragranular blend, roller compacting the intragranular blend to obtain roller compacted granules, blending extragranular components to obtain an extragranular blend, blending the roller compacted granules with the extragranular blend to obtain the final blend, and compressing the final blend to obtain the minitablet.

In one embodiment, the intragranular blending step comprises mixing Compound 1A with intragranular components including, a binder, a sweet diluent, a disintegrant, a wetting agent, a flow agent, a stabilizer, and a high intensity sweetener to obtain the intragranular blend. In one embodiment, the intragranular blending step comprises mixing Compound 1B with intragranular components including, a binder, a sweet diluent, a disintegrant, a wetting agent, a flow agent, a stabilizer, and a high intensity sweetener to obtain the intragranular blend. In one embodiment, the intragranular blend is mixed with a lubricant to obtain a lubricated intragranular blend. In one embodiment, the lubricated intragranular blend is roller compacted to obtain roller compacted granules. The roller compacted granules are blended with an extragranular blend to obtain a final blend, and compressing the final blend to obtain the minitablet.

In one embodiment, the extragranular blending step comprises mixing extragranular components including, a binder, a disintegrant, and a flow agent to obtain the extragranular blend.

In one embodiment, the process for preparing a minitablet comprises: i) blending intragranular components to obtain an intragranular blend, ii) roller compacting the intragranular blend to obtain roller compacted granules, iii) blending extragranular components to obtain an extragranular blend, iv) blending the roller compacted granules with the extragranular blend to obtain a final blend, and v) compressing the final blend to obtain the minitablet, wherein the intragranular blend comprises 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate (Compound 1A), a binder, a sweet diluent, a disintegrant, a wetting agent, a flow agent, a stabilizer, a high intensity sweetener and a lubricant, and the extragranular blend comprises a binder, a disintegrant, and a flow agent.

In one embodiment, the process to prepare a minitablet comprises: i) intragranular blending step comprises blending Compound 1A with intragranular components including, microcrystalline cellulose, hydroxypropyl cellulose, sodium starch glycolate, sodium lauryl sulfate, colloidal SiO₂, mannitol, sucralose, hypromellose acetate succinate to obtain an intragranular blend; ii) blending the intragranular blend with magnesium stearate to obtain a lubricated intragranular blend; iii) roller compacting the lubricated intragranular blend to obtain roller compacted granules; iv) blending the roller compacted granules with an extragranular blend to obtain a final blend, wherein the extragranular blend is obtained by blending extragranular components including, microcrystalline cellulose, sodium starch glycolate and colloidal SiO₂ and magnesium stearate, and v) compressing the final blend to obtain the minitablet.

In one embodiment, the process to prepare a minitablet comprises: i) intragranular blending step comprises blending Compound 1B with intragranular components including, microcrystalline cellulose, hydroxypropyl cellulose, sodium starch glycolate, sodium lauryl sulfate, colloidal SiO₂, mannitol, sucralose, hypromellose acetate succinate to obtain an intragranular blend; ii) blending the intragranular blend with magnesium stearate to obtain a lubricated intragranular blend; iii) roller compacting the lubricated intragranular blend to obtain roller compacted granules; iv) blending the roller compacted granules with an extragranular blend to obtain a final blend, wherein the extragranular blend is obtained by blending extragranular components including, microcrystalline cellulose, sodium starch glycolate and colloidal SiO₂ and magnesium stearate, and v) compressing the final blend to obtain the minitablet.

In one embodiment, the process further comprises filling the minitablets into a capsule.

Examples of the equipment used in the processes for making a tablet provided here include, blenders, such as bin-blender and V-blenders; mills such as comil (e.g. Quadro comil and Freewitt ConiWitt); roller compactors, such as Gerteis, Freund-Vector TFC, Alexanderwerk WP and Fitz Patrick Chilsonator; ribbon mill such as built-in star or pocket granulator, Freewitt OscilloWitt, Fitzmill; tablet press such as Korsch and Killian; encapsulator such as IMA Zanasi and Bosch GKF; weight sorter such as Qualicaps CWI and Bosch KKE.

Any tableting and encapsulating conditions suitable for tablet molding and encapsulation, respectively, can be used. In certain embodiments, the minitablets provided herein are prepared using direct compression process, as well as roller compaction. In certain embodiments, roll force is used such that the tablets are not damaged during the manufacturing process. In certain embodiments, the roller compaction is performed at, for example, from about 1 kN/cm to about 4 kN/cm, in one embodiment, from about 1.5 kN/cm to about 3.5 kN/cm, and in another embodiment, from about 2 kN/cm to about 3 kN/cm. In one embodiment, the roller compaction is performed at about 1.5 kN/cm. In one embodiment, the roller compaction is performed at about 2.5 kN/cm.

The minitablets provided herein exhibit rapid dispersion and dissolution. In one embodiment, in the minitablets provided herein, about 75% or more of Compound 1A dissolves within 15-60 minutes. In one embodiment, in the tablets provided herein, more than 80% of Compound 1A dissolves within 15-60 minutes. In one embodiment, in the minitablets provided herein, more than 85% of Compound 1A dissolves within 15-60 minutes. In one embodiment, in the minitablets provided herein, more than 90% of Compound 1A dissolves within 15-60 minutes. In one embodiment, in the minitablets provided herein, more than 95% of Compound 1A dissolves within 90 minutes. In one embodiment, in the minitablets provided herein, about 75% or more of Compound 1B dissolves within 15-60 minutes. In one embodiment, in the tablets provided herein, more than 80% of Compound 1B dissolves within 15-60 minutes. In one embodiment, in the minitablets provided herein, more than 85% of Compound 1B dissolves within 15-60 minutes. In one embodiment, in the minitablets provided herein, more than 90% of Compound 1B dissolves within 15-60 minutes. In one embodiment, in the minitablets provided herein, more than 95% of Compound 1B dissolves within 90 minutes.

The minitablet provided herein has a good stability during storage. In one embodiment, dissolution of the tablet is not reduced for up to 6 months when storing at temperature of 25 °C at relative humidity of 60%. In one embodiment, dissolution of the minitablet is not reduced for up to 6 months when storing at temperature of 40 °C at relative humidity of 75%. In one embodiment, dissolution of the minitablet is not reduced for up to at least 1 month, at least 2 months, at least 3 months, at least 4 months, at least 6 months, at least 8 months, at least 10 months, or at least 12 months when storing at temperature of 25 °C at relative humidity of 60%. In one embodiment, dissolution of the minitablet is not reduced for up to at least 1 month, at least 2 months, at least 3 months, at least 4 months, at least 6 months, at least 8 months, at least 10 months, or at least 12 months when storing at temperature of 40 °C at relative humidity of 75%.

In one embodiment, the minitablet composition provided herein comprises Compound 1D at the time of manufacture of the minitablet in an amount of ≤ 10%, ≤ 9%.or ≤ 8% based on the total weight of Compound 1B in the minitablet. In one embodiment, the minitablet composition provided herein comprises Compound 1D at the time of manufacture of the minitablet in an amount of ≤ 8% based on the total weight of Compound 1B in the minitablet. In one embodiment, the minitablet composition provided herein comprises Compound 1D at the time of manufacture of the minitablet in an amount of about 8% based on the total weight of Compound 1B in the minitablet.

In one embodiment, the minitablet formulations provided herein show minor increase in the amorphous content (Compound 1D) upon storage. In one embodiment, the minitablet formulations provided herein comprise about 10-11% Compound 1D based on the total weight of Compound 1B in the minitablet upon storage for about 1 month. In one embodiment, the minitablet formulations provided herein comprise about 10-11% Compound 1D based on the total weight of Compound 1B in the minitablet upon storage for about 1 month at about 5 °C. In one embodiment, the minitablet formulations provided herein comprise about 10-11% Compound 1D based on the total weight of Compound 1B in the minitablet upon storage for about 1 month at about 25 °C at 60% relative humidity. In one embodiment, the minitablet formulations provided herein comprise about 10-11% Compound 1D based on the total weight of Compound 1B in the minitablet upon storage for about 1 month at about 40 °C at 75% relative humidity.

In one embodiment, the minitablet formulations provided herein comprise about 11-12% Compound 1D based on the total weight of Compound 1B in the minitablet upon storage for about 3-4 months. In one embodiment, the minitablet formulations provided herein comprise about 11-12% Compound 1D based on the total weight of Compound 1B in the minitablet upon storage for about 3-4 months at about 5 °C. In one embodiment, the minitablet formulations provided herein comprise about 11-12% Compound 1D based on the total weight of Compound 1B in the minitablet upon storage for about 3-4 months at about 25 °C at 60% relative humidity. In one embodiment, the minitablet formulations provided herein comprise about 11-12% Compound 1D based on the total weight of Compound 1B in the minitablet upon storage for about 3-4 months at about 40 °C at 75% relative humidity.

In one embodiment, the minitablet formulations provided herein comprise about 12-14% Compound 1D based on the total weight of Compound 1B in the minitablet upon storage for about 6 months. In one embodiment, the minitablet formulations provided herein comprise about 12-14% Compound 1D based on the total weight of Compound 1B in the minitablet upon storage for about 6 months at about 5 °C. In one embodiment, the minitablet formulations provided herein comprise about 12-14% Compound 1D based on the total weight of Compound 1B in the minitablet upon storage for about 6 months at about 25 °C at 60% relative humidity. In one embodiment, the minitablet formulations provided herein comprise about 12-14% Compound 1D based on the total weight of Compound 1B in the minitablet upon storage for about 6 months at about 40 °C at 75% relative humidity.

### 6. Methods of Use

Any references to methods of treatment by therapy or surgery or in vivo diagnosis methods of this description is to be interpreted as a reference to compounds, pharmaceutical compositions and medicaments of the present invention for use in those methods.

The pediatric formulations provided herein are useful for treating a disease selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma, lessen the severity of the disease/disorder (i.e., a disease selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma, each characterized by the presence of a mutant allele of IDH2.

Provided herein are pediatric formulations for use in the methods of treating a disease provided herein.

In one embodiment, provided herein is a method of treating and preventing a disease or condition, comprising the administration of a pediatric formulation comprising Compound 1A, wherein the disease is selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma, lessen the severity of the disease/disorder (i.e., a disease selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma, each characterized by the presence of a mutant allele of IDH2.

In one embodiment, provided herein is a method of treating AML selected from newly diagnosed AML, previously untreated AML, AML arising from MDS, AML arising from antecedent hematologic disorder (AHD) and AML arising after exposure to genotoxic injury. In certain embodiments, the genotoxic injury is resulting from radiation and/or chemotherapy. In one embodiment, provided herein is a method of treating AML arising after exposure to genotoxic injury resulting from radiation and/or chemotherapy), each characterized by the presence of a mutant allele of IDH2.

In one embodiment, provided herein is a method of treating newly diagnosed AML characterized by the presence of a mutant allele of IDH2.

In one embodiment, provided herein is a method of treating previously untreated AML characterized by the presence of a mutant allele of IDH2.

In one embodiment, provided herein is a method of treating AML arising from MDS characterized by the presence of a mutant allele of IDH2.

In one embodiment, provided herein is a method of treating AML arising from AHD characterized by the presence of a mutant allele of IDH2.

In one embodiment, provided herein is a method of treating AML arising after exposure to genotoxic injury characterized by the presence of a mutant allele of IDH2.

In one embodiment, provided herein is a method of treating myeloproliferative neoplasm (MPN).

In one aspect of this embodiment, the mutant IDH2 has an R140X mutation. In another aspect of this embodiment, the R140X mutation is a R140Q mutation. In another aspect of this embodiment, the R140X mutation is a R140W mutation. In another aspect of this embodiment, the R140X mutation is a R140L mutation. In another aspect of this embodiment, the mutant IDH2 has an R172X mutation. In another aspect of this embodiment, the R172X mutation is a R172K mutation. In another aspect of this embodiment, the R172X mutation is a R172G mutation. A cancer selected from AML, MDS, CMML, or lymphoma (e.g., T-cell lymphoma) can be analyzed by sequencing cell samples to determine the presence and specific nature of (e.g., the changed amino acid present at) a mutation at amino acid 140 and/or 172 of IDH2.

Without being bound by any theory, it is believed that mutant alleles of IDH2 wherein the IDH2 mutation results in a new ability of the enzyme to catalyze the NADPH-dependent reduction of α-ketoglutarate to R(-)-2-hydroxyglutarate, and in particular R140Q and/or R172K mutations of IDH2, characterize a subset of all types of cancers described herein, without regard to their cellular nature or location in the body. Thus, the methods of one aspect are useful to treat a hematologic cancer selected from AML, MDS, CMML, or lymphoma (e.g., T-cell lymphoma) or solid tumor selected from glioma, melanoma, chondrosarcoma, cholangiocarcinoma (e.g., glioma) and AITL, that is characterized by the presence of a mutant allele of IDH2 imparting such activity and in particular an IDH2 R140Q and/or R172K mutation.

In one embodiment, the efficacy of treatment is monitored by measuring the levels of 2HG in the subject. Typically levels of 2HG are measured prior to treatment, wherein an elevated level is indicated for the use of Compound 1 to treat the disease selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma. Once the elevated levels are established, the level of 2HG is determined during the course of and/or following termination of treatment to establish efficacy. In certain embodiments, the level of 2HG is only determined during the course of and/or following termination of treatment. A reduction of 2HG levels during the course of treatment and following treatment is indicative of efficacy. Similarly, a determination that 2HG levels are not elevated during the course of or following treatment is also indicative of efficacy. Typically, the these 2HG measurements will be utilized together with other well-known determinations of efficacy of cancer treatment, such as reduction in number and size of tumors and/or other cancer-associated lesions, improvement in the general health of the subject, and alterations in other biomarkers that are associated with cancer treatment efficacy.

2HG can be detected in a sample by the methods of PCT Publication No. WO 2013/102431 and US Publication No. US 2013/0190287, or by analogous methods.

In one embodiment 2HG is directly evaluated.

In another embodiment a derivative of 2HG formed in process of performing the analytic method is evaluated. By way of example such a derivative can be a derivative formed in MS analysis. Derivatives can include a salt adduct, *e.g.,* a Na adduct, a hydration variant, or a hydration variant which is also a salt adduct, e.g., a Na adduct, e.g., as formed in MS analysis.

In another embodiment a metabolic derivative of 2HG is evaluated. Examples include species that build up or are elevated, or reduced, as a result of the presence of 2HG, such as glutarate or glutamate that will be correlated to 2HG, *e.g.,* R-2HG.

Exemplary 2HG derivatives include dehydrated derivatives such as the compounds provided below or a salt adduct thereof:

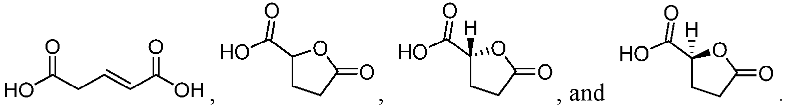

In one embodiment the cancer selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma is a tumor wherein at least 30, 40, 50, 60, 70, 80 or 90% of the tumor cells carry an IDH2 mutation, and in particular an IDH2 R140Q, R140W, or R140L and/or R172K or R172G mutation, at the time of diagnosis or treatment.

In one embodiment, the cancer to be treated is AML. In some embodiments, the AML is relapsed and/or primary refractory. In some embodiments, the AML is relapsed and/or refractory. In other embodiments, the AML is previously untreated. In one embodiment, the AML is newly diagnosed AML.

In another embodiment, the cancer to be treated is MDS with refractory anemia with excess blasts (subtype RAEB-1 or RAEB-2). In other embodiments, the MDS is previously untreated. In one embodiment, the MDS is newly diagnosed MDS.

In another embodiment, the cancer to be treated is relapsed and/or primary refractory CMML.

In certain embodiments, the pediatric formulations provided herein are for treating a hematologic malignancy characterized by the presence of a mutant allele of IDH2 and the absence of a mutant allele of FLT3 and/or a mutant allele of NRAS. Exemplary methods for treating a hematologic malignancy characterized by the presence of a mutant allele of IDH2 and the absence of a mutant allele of FLT3 and/or a mutant allele of NRAS by administering Compound 1A are described in US Patent Nos. 10,137,130 and 10,188,656.

In one embodiment, the pediatric formulations provided herein are for treating a hematologic malignancy characterized by the presence of a mutant allele of IDH2 and the absence of a mutant allele of FLT3. In one embodiment, the hematologic malignancy is an advanced hematologic malignancy. In one embodiment, the hematologic malignancy is AML. In some embodiments, the AML is relapsed and/or refractory.

In one embodiment, the pediatric formulations provided herein are for treating a hematologic malignancy characterized by the presence of a mutant allele of IDH2 and the absence of a mutant allele of NRAS. In one embodiment, the hematologic malignancy is an advanced hematologic malignancy. In one embodiment, the hematologic malignancy is AML. In some embodiments, the AML is relapsed and/or refractory.

In one embodiment, provided herein are methods of treating a hematologic malignancy by administering a pediatric formulation comprising Compound 1A in combination with a therapeutically effective amount of one or more compounds that target a FLT3 pathway, wherein the hematologic malignancy is characterized by the presence of a mutant allele of IDH2 and a mutant allele of FLT3, for example FLT3-ITD or FLT3-KDM. In one embodiment, the hematologic malignancy is an advanced hematologic malignancy. In one embodiment, the hematologic malignancy is AML. In some embodiments, the AML is relapsed and/or refractory.

In one embodiment, provided herein is a method of treating hematologic malignancies, such as AML, MDS, CMML, myeloid sarcoma, multiple myeloma, lymphoma (e.g., T-cell lymphoma or B-cell lymphoma), AITL or blastic plasmacytoid dendritic cell neoplasm, each characterized by the presence of a mutant allele of IDH2 and the absence of a mutant allele of FLT3, comprising administering a pediatric formulationcomprising Compound 1A. In one embodiment, the hematologic malignancy is an advanced hematologic malignancy. In one embodiment, the hematologic malignancy is AML. In some embodiments, the AML is relapsed and/or refractory.

In one embodiment, provided herein is a method of treating hematologic malignancies, such as AML, MDS, CMML, myeloid sarcoma, multiple myeloma, lymphoma (e.g., T-cell lymphoma or B-cell lymphoma), AITL or blastic plasmacytoid dendritic cell neoplasm, each characterized by the presence of a mutant allele of IDH2 and a mutant allele of FLT3, for example FLT3-ITD, comprising administering a pediatric formulation comprising Compound 1A in combination with a therapeutically effective amount of one or more compounds that target a FLT3 pathway. Exemplary FLT3 inhibitors are described elsewhere herein. In one embodiment, the hematologic malignancy is an advanced hematologic malignancy. In one embodiment, the hematologic malignancy is AML. In some embodiments, the AML is relapsed and/or refractory.

In one embodiment, provided herein are methods of treating solid tumors by administering a pediatric formulation comprising Compound 1A, wherein the solid tumor is characterized by the presence of a mutant allele of IDH2 and the absence of a mutant allele of FLT3. In one embodiment, the solid tumor is an advanced solid tumor. In some embodiments, the AML is relapsed and/or refractory.

In one embodiment, provided herein are methods of treating solid tumors by administering to a subject a pediatric formulation comprising Compound 1A in combination with a therapeutically effective amount of one or more compounds that target a FLT3 pathway, wherein the solid tumor is characterized by the presence of a mutant IDH2 and a mutant allele of FLT3, for example FLT3-ITD. In one embodiment, the solid tumor is an advanced solid tumor.

In one embodiment, provided herein is a method of treating solid tumors, such as glioma, melanoma, chondrosarcoma, or cholangiocarcinoma(e.g., glioma), or treating AITL, each characterized by the presence of a mutant allele of IDH2 and the absence of a mutant allele of FLT3, comprising administering to a subject a pediatric formulation provided herein.

In one embodiment, provided herein is a method of treating solid tumors, such as glioma, melanoma, chondrosarcoma, or cholangiocarcinoma (e.g., glioma), or treating AITL, each characterized by the presence of a mutant allele of IDH2 and a mutant allele of FLT3, in a subject comprising administering a pediatric formulation comprising Compound 1A in combination with a therapeutically effective amount of one or more compounds that target a FLT3 pathway. Exemplary FLT3 inhibitors are described elsewhere herein.

In one embodiment, provided herein is a method of treating a hematologic malignancy by administering a pediatric formulation comprising Compound 1A, wherein the hematologic malignancy is characterized by the presence of a mutant allele of IDH2 and the absence of a mutant allele of NRAS. In one embodiment, the hematologic malignancy is an advanced hematologic malignancy.

In one embodiment, provided herein is a method of treating a hematologic malignancy by administering a pediatric formulation comprising Compound 1A in combination with a therapeutically effective amount of one or more compounds that target RAS pathways, wherein the hematologic malignancy is characterized by the presence of a mutant allele of IDH2 and a mutant allele of NRAS. In one embodiment, the hematologic malignancy is an advanced hematologic malignancy.

In one embodiment, provided herein is a method of treating a hematologic malignancy, such as AML, MDS, CMML, myeloid sarcoma, multiple myeloma, lymphoma (e.g., T-cell lymphoma or B-cell lymphoma), AITL or blastic plasmacytoid dendritic cell neoplasm, each characterized by the presence of a mutant allele of IDH2 and the absence of a mutant allele of NRAS, comprising administering a pediatric formulation comprising Compound 1A. In one embodiment, the hematologic malignancy is an advanced hematologic malignancy.

In one embodiment, provided herein is a method of treating hematologic malignancies, such as AML, MDS, CMML, myeloid sarcoma, multiple myeloma, lymphoma (e.g., T-cell lymphoma or B-cell lymphoma), AITL or blastic plasmacytoid dendritic cell neoplasm, each characterized by the presence of a mutant allele of IDH2 and a mutant allele of NRAS comprising administering a pediatric formulation comprising Compound 1A in combination with a therapeutically effective amount of one or more compounds that target RAS pathways. In one embodiment, a pediatric formulation comprising Compound 1A is administered to the subject in combination with a therapeutically effective amount of a MEK kinase inhibitor. Exemplary MEK kinase inhibitors are described elsewhere herein. In one embodiment, the hematologic malignancy is an advanced hematologic malignancy.

In one embodiment, provided herein are methods of treating solid tumors by administering a pediatric formulation comprising Compound 1A, wherein the solid tumor is characterized by the presence of a mutant allele of IDH2 and the absence of a mutant allele of NRAS. In one embodiment, the solid tumor is an advanced solid tumor.

In one embodiment, provided herein are methods of treating solid tumors by administering a pediatric formulation comprising Compound 1A in combination with a therapeutically effective amount of one or more compounds that target RAS pathways, wherein the solid tumor is characterized by the presence of a mutant IDH2 and a mutant allele of NRAS. In one embodiment, the solid tumor is an advanced solid tumor.

In one embodiment, provided herein is a method of treating solid tumors, such as glioma, melanoma, chondrosarcoma, or cholangiocarcinoma (e.g., glioma), or treating angioimmunoblastic T-cell lymphoma (AITL), each characterized by the presence of a mutant allele of IDH2 and the absence of a mutant allele of NRAS, comprising administering a pediatric formulation comprising Compound 1A.

In one embodiment, provided herein is a method of treating solid tumors, such as glioma, melanoma, chondrosarcoma, or cholangiocarcinoma (e.g., glioma), or treating angioimmunoblastic T-cell lymphoma (AITL), each characterized by the presence of a mutant allele of IDH2 and a mutant allele of NRAS, comprising administering a pediatric formulation comprising Compound 1A in combination with a therapeutically effective amount of one or more compounds that target RAS pathways.

In one embodiment, provided herein are methods of treating MPN in a subject comprising administering to the subject a pediatric formulation comprising Compound 1A in combination with a therapeutically effective amount of a JAK2 inhibitor, wherein the subject harbors a mutant allele of IDH2 and a mutant allele of JAK2. Exemplary JAK2 inhibitors are described elsewhere herein.

In certain embodiments, provided herein is a method of treating a high risk MPN in a subject comprising administering to the subject a pediatric formulation comprising Compound 1A in combination with a therapeutically effective amount of a JAK2 inhibitor, wherein the subject harbors a mutant allele of IDH2 and a mutant allele of JAK2.

In one embodiment, provided herein are methods of treating AML in a subject comprising administering to the subject a pediatric formulation comprising Compound 1A in combination with a therapeutically effective amount of a JAK2 inhibitor, wherein the subject harbors a mutant allele of IDH2 and a mutant allele of JAK2. In some embodiments, the AML is relapsed and/or refractory.

In certain embodiments, the mutant allele of IDH2 is mIDH2-R140 or mIDH2-R172.

In certain embodiments, the mutant allele of IDH2 is mIDH2-R140Q, mIDH2-R140W, mIDH2-R140L, mIDH2-R172K, or mIDH2-R172G.

In certain embodiments, the mutant allele of JAK2 is mJAK2-V617F.

In certain embodiments, the pediatric formulations provided herein are for treating MDS characterized by the presence of a mutant allele of IDH2 and a mutant allele of at least one second gene, wherein the second gene is selected from the group consisting of ASXL1 and SRSF2. In certain embodiments, the pediatric formulations provided herein are for treating MDS characterized by the presence of a mutant allele of IDH2 and the absence of a mutant allele of at least one other gene, wherein the other gene is selected from the group consisting of KRAS, TP53, SETBP1, and U2AF1. In certain embodiments, the pediatric formulations provided herein are for treating MDS characterized by the presence of a mutant allele of IDH2 and the absence of a mutant allele of at least one other gene, wherein the other gene is selected from the group consisting of KRAS, TP53, SETBP1, U2AF1, TCF3, STAG2, NRAS, JAK2 and BRAF. Exemplary methods of treating MDS characterized by the presence of a mutant allele of IDH2 by administering Compound 1A are described in US 2018/0042930-A.

In one embodiment, prior to and/or after treatment with a pediatric formulationcomprising Compound 1A provided herein, the method further comprises the step of evaluating the growth, size, weight, invasiveness, stage and/or other phenotype of the cancer selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma.

In one embodiment, prior to and/or after treatment with a composition provided herein, the method further comprises the step of evaluating the IDH2 genotype of the cancer selected from AML, MDS, CMML, myeloid sarcoma, multiple myeloma, lymphoma (e.g., T-cell lymphoma or B-cell lymphoma), AITL, blastic plasmacytoid dendritic cell neoplasm, MPN, glioma, melanoma, chondrosarcoma, and cholangiocarcinoma. This may be achieved by ordinary methods in the art, such as DNA sequencing, immuno analysis, and/or evaluation of the presence, distribution or level of 2HG.

In one embodiment, prior to and/or after treatment with a composition provided herein, the method further comprises the step of determining the 2HG level in the subject. This may be achieved by spectroscopic analysis, *e.g.,* magnetic resonance-based analysis, *e.g.,* MRI and/or MRS measurement, sample analysis of bodily fluid, such as blood, plasma, urine, or spinal cord fluid analysis, or by analysis of surgical material, e.g., by mass-spectroscopy (e.g. LC-MS, GC-MS).

In one embodiment, the pediatric formulation comprising Compound 1A is for use in any of the above described methods. In one embodiment, the pediatric formulation comprising Compound 1B is for use in any of the above described methods.

In certain embodiments, the pediatric formulation provided herein for methods described herein is administered once daily.

In one embodiment, the pediatric formulation comprising Compound 1A is administered to deliver a dose of about 20 mg, about 40 mg or about 60 mg of Compound 1C for use in any of the above described methods. In one embodiment, the pediatric formulation comprising Compound 1B is administered to deliver a dose of about 20 mg, about 40 mg or about 60 mg of Compound 1C for use in any of the above described methods.

In certain embodiments, the pediatric formulation provided herein is administered to a pediatric patient in cycles (e.g., daily administration for one week, then a rest period with no administration for up to three weeks). Cycling therapy involves the administration of an active agent for a period of time, followed by a rest for a period of time, and repeating this sequential administration. Cycling therapy can reduce the development of resistance, avoid or reduce the side effects, and/or improves the efficacy of the treatment.

In one embodiment, a method provided herein comprises administering the pediatric formulation provided herein in 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or greater than 40 cycles. In certain embodiments, the pediatric formulation provided herein for methods described herein is administered for 1 to 25 cycles. In one embodiment, the median number of cycles administered in a group of pediatric patients is about 1. In one embodiment, the median number of cycles administered in a group of pediatric patients is about 2. In one embodiment, the median number of cycles administered in a group of patients is about 3. In one embodiment, the median number of cycles administered in a group of pediatric patients is about 4. In one embodiment, the median number of cycles administered in a group of pediatric patients is about 5. In one embodiment, the median number of cycles administered in a group of pediatric patients is about 6. In one embodiment, the median number of cycles administered in a group of pediatric patients is about 7. In one embodiment, the median number of cycles administered in a group of pediatric patients is about 8. In one embodiment, the median number of cycles administered in a group of patients is about 9. In one embodiment, the median number of cycles administered in a group of pediatric patients is about 10. In one embodiment, the median number of cycles administered in a group of pediatric patients is about 11. In one embodiment, the median number of cycles administered in a group of pediatric patients is about 12. In one embodiment, the median number of cycles administered in a group of pediatric patients is about 13. In one embodiment, the median number of cycles administered in a group of pediatric patients is about 14. In one embodiment, the median number of cycles administered in a group of pediatric patients is about 15. In one embodiment, the median number of cycles administered in a group of pediatric patients is about 16. In one embodiment, the median number of cycles administered in a group of pediatric patients is about 17. In one embodiment, the median number of cycles administered in a group of pediatric patients is about 18. In one embodiment, the median number of cycles administered in a group of pediatric patients is about 19. In one embodiment, the median number of cycles administered in a group of pediatric patients is about 20. In one embodiment, the median number of cycles administered in a group of pediatric patients is about 21. In one embodiment, the median number of cycles administered in a group of pediatric patients is about 22. In one embodiment, the median number of cycles administered in a group of pediatric patients is about 23. In one embodiment, the median number of cycles administered in a group of pediatric patients is about 24. In one embodiment, the median number of cycles administered in a group of pediatric patients is about 25. In one embodiment, the median number of cycles administered in a group of pediatric patients is about 26. In one embodiment, the median number of cycles administered in a group of pediatric patients is about 27. In one embodiment, the median number of cycles administered in a group of pediatric patients is about 28. In one embodiment, the median number of cycles administered in a group of pediatric patients is about 29. In one embodiment, the median number of cycles administered in a group of pediatric patients is about 30. In one embodiment, the median number of cycles administered in a group of pediatric patients is greater than about 30 cycles.

In certain embodiments, treatment cycles comprise multiple doses of the pediatric formulation provided herein administered to a pediatric patient in need thereof over multiple days (*e.g.,* 1, 2, 3, 4, 5, 6, 7, 8, 9, 10, 11, 12, 13, 14, or greater than 14 days), optionally followed by treatment dosing holidays (*e.g*., 1, 2, 3, 4, 5, 6, 7, 8, 9, 10, 11, 12, 13, 14, 15, 16, 17, 18, 19, 20, 21, 22, 23, 24, 25, 26, 27, 28, or greater than 28 days).

In certain embodiments, the pediatric formulation provided herein is administered in one or more 28 day cycles in the methods described herein. In certain embodiments, the pediatric formulation provided herein is administered in a 28 day cycle in the methods described herein.

In certain embodiments, the pediatric formulation provided herein is administered orally in the methods described herein. In certain embodiments, the pediatric formulation provided herein is administered by sprinkling the minitablets onto soft foods, such as yogurt, gelatin snack, and pudding.

In certain embodiments, the pediatric formulation provided herein is administered once daily orally in the methods described herein.

In certain embodiments, the pediatric patient for the methods provided herein is a patient 21 years or younger, in certain embodiments, a patient 18 years or younger, in certain embodiments, a patient 16 years or younger, in certain embodiments, a patient 14 years or younger, in certain embodiments, a patient 12 years or younger, in certain embodiments, a patient 10 years or younger, or in certain embodiments, a patient 8 years or younger, or in certain embodiments, a patient 6 years or younger. In certain embodiments, a pediatric patient is a patient 12-18 years old. In certain embodiments, a pediatric patient is a patient 6-12 years old. In certain embodiments, a pediatric patient is a patient 2-6 years old.

### 7. Combination Therapy

In certain embodiments, the pediatric formulations provided herein are used with an additional cancer therapeutic agent or an additional cancer treatment. In certain embodiments, provided herein are pediatric formulations for use in the methods provided herein, wherein the methods further comprise administering an additional cancer therapeutic agent or comprise an additional cancer treatment. Exemplary additional cancer therapeutic agents and additional cancer treatments are described in US Patent Nos. 9,732,062; 10,188,656; 10,188,656; 10,137,130; and US Application Publication Nos. 2018/0303840-A1 and 2018/0311249-A1; and International Application Publication No. WO 2018/204787.

In certain embodiments, additional cancer therapeutic agents include for example, chemotherapy, targeted therapy, antibody therapies, immunotherapy, and hormonal therapy. In certain embodiments, additional cancer treatments include, for example: surgery, and radiation therapy. Examples of each of these treatments are provided below.

In some embodiments, the additional cancer therapeutic agent is a chemotherapy agent. Examples of chemotherapeutic agents used in cancer therapy include, for example, antimetabolites (e.g., folic acid, purine, and pyrimidine derivatives), alkylating agents (e.g., nitrogen mustards, nitrosoureas, platinum, alkyl sulfonates, hydrazines, triazenes, aziridines, spindle poison, cytotoxic agents, topoisomerase inhibitors and others), and hypomethylating agents (e.g., decitabine (5-aza-deoxycytidine), zebularine, isothiocyanates, azacitidine (5-azacytidine), 5-flouro-2'-deoxycytidine, 5,6-dihydro-5-azacytidine and others). Exemplary agents include Aclarubicin, Actinomycin, Alitretinoin, Altretamine, Aminopterin, Aminolevulinic acid, Amrubicin, Amsacrine, Anagrelide, Arsenic trioxide, Asparaginase, Atrasentan, Belotecan, Bexarotene, bendamustine, Bleomycin, Bortezomib, Busulfan, Camptothecin, Capecitabine, Carboplatin, Carboquone, Carmofur, Carmustine, Celecoxib, Chlorambucil, Chlormethine, Cisplatin, Cladribine, Clofarabine, Crisantaspase, Cyclophosphamide, Cytarabine, Dacarbazine, Dactinomycin, Daunorubicin, Decitabine, Demecolcine, Docetaxel, Doxorubicin, Efaproxiral, Elesclomol, Elsamitrucin, Enocitabine, Epirubicin, Estramustine, Etoglucid, Etoposide, Floxuridine, Fludarabine, Fluorouracil (5FU), Fotemustine, Gemcitabine, Gliadel implants, Hydroxycarbamide, Hydroxyurea, Idarubicin, Ifosfamide, Irinotecan, Irofulven, Ixabepilone, Larotaxel, Leucovorin, Liposomal doxorubicin, Liposomal daunorubicin, Lonidamine, Lomustine, Lucanthone, Mannosulfan, Masoprocol, Melphalan, Mercaptopurine, Mesna, Methotrexate, Methyl aminolevulinate, Mitobronitol, Mitoguazone, Mitotane, Mitomycin, Mitoxantrone, Nedaplatin, Nimustine, Oblimersen, Omacetaxine, Ortataxel, Oxaliplatin, Paclitaxel, Pegaspargase, Pemetrexed, Pentostatin, Pirarubicin, Pixantrone, Plicamycin, Porfimer sodium, Prednimustine, Procarbazine, Raltitrexed, Ranimustine, Rubitecan, Sapacitabine, Semustine, Sitimagene ceradenovec, Strataplatin, Streptozocin, Talaporfin, Tegafur uracil, Temoporfin, Temozolomide, Teniposide, Tesetaxel, Testolactone, Tetranitrate, Thiotepa, Tiazofurine, Tioguanine, Tipifarnib, Topotecan, Trabectedin, Triaziquone, Triethylenemelamine, Triplatin, Tretinoin, Treosulfan, Trofosfamide, Uramustine, Valrubicin, Verteporfin, Vinblastine, Vincristine, Vindesine, Vinflunine, Vinorelbine, Vorinostat, Zorubicin, and other cytostatic or cytotoxic agents described herein.

Because some drugs work better together than alone, two or more drugs are often given at the same time. Often, two or more chemotherapy agents are used as combination chemotherapy.

In some embodiments, the additional cancer therapeutic agent is a differentiation agent. Such differentiation agent includes retinoids (such as all-trans-retinoic acid (ATRA), 9-cis retinoic acid, 13-cis-retinoic acid (13-cRA) and 4-hydroxy-phenretinamide (4-HPR)); arsenic trioxide; histone deacetylase inhibitors HDACs (such as azacytidine (Vidaza) and butyrates (e.g., sodium phenylbutyrate)); hybrid polar compounds (such as hexamethylene bisacetamide ((HMBA)); vitamin D; and cytokines (such as colony-stimulating factors including G-CSF and GM-CSF, and interferons).

In some embodiments the additional cancer therapeutic agent is a targeted therapy agent. Targeted therapy constitutes the use of agents specific for the deregulated proteins of cancer cells. Small molecule targeted therapy drugs are generally inhibitors of enzymatic domains on mutated, overexpressed, or otherwise critical proteins within the cancer cell. Prominent examples are the tyrosine kinase inhibitors such as Axitinib, Bosutinib, Cediranib, dasatinib, erlotinib, imatinib, gefitinib, lapatinib, Lestaurtinib, Nilotinib, Semaxanib, Sorafenib, Sunitinib, and Vandetanib, and also cyclin dependent kinase inhibitors such as Alvocidib and Seliciclib. Monoclonal antibody therapy is another strategy in which the therapeutic agent is an antibody which specifically binds to a protein on the surface of the cancer cells. Examples include the anti HER2/neu antibody trastuzumab (HERCEPTIN^{®}) typically used in breast cancer, and the anti CD20 antibody rituximab and Tositumomab typically used in a variety of B cell malignancies. Other exemplary antibodies include Cetuximab, Panitumumab, Trastuzumab, Alemtuzumab, Bevacizumab, Edrecolomab, and Gemtuzumab. Exemplary fusion proteins include Aflibercept and Denileukin diftitox. In some embodiments, the targeted therapy can be used in combination with a compound described herein, e.g., a biguanide such as metformin or phenformin, preferably phenformin.

Targeted therapy can also involve small peptides as "homing devices" which can bind to cell surface receptors or affected extracellular matrix surrounding the tumor. Radionuclides which are attached to these peptides (e.g., RGDs) eventually kill the cancer cell if the nuclide decays in the vicinity of the cell. An example of such therapy includes BEXXAR^{®}.

In some embodiments, the additional cancer therapeutic agent is an immunotherapy agent. Cancer immunotherapy refers to a diverse set of therapeutic strategies designed to induce the subject's own immune system to fight the tumor. Contemporary methods for generating an immune response against tumors include intravesicular BCG immunotherapy for superficial bladder cancer, and use of interferons and other cytokines to induce an immune response in renal cell carcinoma and melanoma subjects.

Allogeneic hematopoietic stem cell transplantation can be considered a form of immunotherapy, since the donor's immune cells will often attack the tumor in a graft versus tumor effect. In some embodiments, the immunotherapy agents can be used in combination with a compound or composition described herein.

In some embodiments, the additional cancer therapeutic agent is a hormonal therapy agent. The growth of some cancers can be inhibited by providing or blocking certain hormones. Common examples of hormone sensitive tumors include certain types of breast and prostate cancers. Removing or blocking estrogen or testosterone is often an important additional treatment. In certain cancers, administration of hormone agonists, such as progestogens may be therapeutically beneficial. In some embodiments, the hormonal therapy agents can be used in combination with a compound or a composition described herein.

Other possible additional therapeutic modalities include imatinib, gene therapy, peptide and dendritic cell vaccines, synthetic chlorotoxins, and radiolabeled drugs and antibodies.

In one embodiment, the compositions provided herein are used for treatment of AML in combination with an AML induction and consolidation therapy. In one embodiment, the AML induction therapy is a combination of cytarabine and daunorubicin. In one embodiment, the AML induction therapy is a combination of cytarabine and idarubicin.

In one embodiment, the AML consolidation therapy is cytarabine. In one embodiment, the AML consolidation therapy is a combination of mitoxantrone and etoposide.

In one embodiment, the compositions provided herein are used in combination with one or more DNA demethylating agents. In one embodiment, the DNA demethylating agent is a cytidine analog. In certain embodiments, the cytidine analog is azacitidine or 5-aza-2'-deoxycytidine (decitabine). In certain embodiments, the cytidine analog is azacitidine. In certain embodiments, the cytidine analog is 5-aza-2'-deoxycytidine (decitabine). In certain embodiments, the cytidine analog is, for example: 1-β-D-arabinofuranosylcytosine (cytarabine or ara-C); pseudoiso-cytidine (psi ICR); 5-fluoro-2'-deoxycytidine (FCdR); 2'-deoxy-2',2'-difluorocytidine (gemcitabine); 5-aza-2'-deoxy-2',2'-difluorocytidine; 5-aza-2'-deoxy-2'-fluorocytidine; 1-β-D-ribofuranosyl-2(1H)-pyrimidinone (zebularine); 2',3'-dideoxy-5-fluoro-3'-thiacytidine (emtriva); 2'-cyclocytidine (ancitabine); 1-β-D-arabinofuranosyl-5-azacytosine (fazarabine or ara-AC); 6-azacitidine (6-aza-CR); 5,6-dihydro-5-azacitidine (dH-aza-CR); N⁴ pentyloxy-carbonyl-5'-deoxy-5-fluorocytidine (capecitabine); N⁴ octadecyl-cytarabine; or elaidic acid cytarabine. In certain embodiments, the cytidine analogs include any compound which is structurally related to cytidine or deoxycytidine and functionally mimics and/or antagonizes the action of cytidine or deoxycytidine.

In one embodiment, the compositions provided herein are used in combination with azacitidine.

In one embodiment, the compositions provided herein are used in combination with a FLT3 inhibitor. In one embodiment, the FLT3 inhibitor is selected from quizartinib (AC220), sunitinib (SU11248), sorafenib (BAY 43-9006), midostaurin (PKC412), crenolanib (CP-868596), PLX3397, E6201, AKN-028, ponatinib (AP24534), ASP2215, KW-2449, famitinib and DCC-2036.

In one embodiment, the compositions provided herein are used in combination with MEK kinase inhibitor. In one embodiment, the MEK kinase is selected from trametinib, selumetinib, binimetinib, PD-325901, cobimetinib, CI-1040 and PD035901.

In one embodiment, the compositions provided herein are used in combination with a JAK inhibitor. In one embodiment, the compositions provided herein are used in combination with a JAK2 inhibitor. In one embodiment, the JAK2 inhibitor is selected from INCB018424 (ruxolitinib), TG101348, CYT387, AZD1480, SB1518 (pacritinib), XL019, NCB0-16562, NVP-BSK805, R723, hydroxycarbamide, SAR302503, CP-690,550 (tasocitinib) and INCB16562. In one embodiment, the compositions provided herein are used in combination with ruxolitinib.

In one embodiment, the compositions provided herein are used in combination with a second agent selected from mercaptopurine, methotrexate, L-asparaginase, vincristine, vindesine, doxorubicin, prednisone, daunorubicin, idarubicin, 6-thioguanine, dexamethasone, 2-chloro-2-deoxyadenosine, an antibody and a kinase inhibitor. In one embodiment, the compositions provided herein are used in combination with a second agent selected from dexamethasone, 2-chloro-2-deoxyadenosine, an antibody and a kinase inhibitor. In one embodiment, the compositions provided herein are used in combination with a second agent selected from dexamethasone, 2-chloro-2-deoxyadenosine, gemtuzumab ozogamicin (GO) and sorafenib.

It is understood that the foregoing detailed description and accompanying examples are merely illustrative, and are not to be taken as limitations upon the scope of the subject matter. Various changes and modifications to the disclosed embodiments will be apparent to those skilled in the art. Such changes and modifications, including without limitation those relating to the methods of use provided herein, may be made without departing from the spirit and scope thereof. Patents, patent publications, and other publications referenced herein are incorporated by reference.

### EXAMPLES

The embodiments described below are intended to be merely exemplary. The scope of the claimed subject-matter is defined by the appended claims.

The following abbreviations are used:

### Abbreviations:

ALT Alanine aminotransferase
ANC Absolute neutrophil count
API Active Pharmaceutical Ingredient
AST Aspartate aminotransferase
AUC Area under the curve
CNS Central nervous system
CR Complete response
CRC Colorectal carcinoma
CRc Cytogenetic complete response
CRi Complete response with incomplete hematological recovery
CRM continual reassessment method
CRp Complete response without platelet recovery
CSF Cerebrospinal fluid
CTCAE Common terminology criteria for adverse events
CU Content Uniformity
DL Drug Load
DLI Donor lymphocyte infusion
DLT Dose limiting toxicity
DOR Duration of response
DP Drug Product
DS Drug Substance
ECG Electrocardiogram
FeSSIF Fed state simulating intestinal fluid
FaSSIF Fstate simulating intestinal fluid
GFI Glomerular filtration rate
GI Gastrointestinal
GVHD Graft-versus-host disease
HDPE High-Density Polyethylene
2HG 2-hydroxygluturate
HGG High-grade glioma
INRC International neuroblastoma response criteria
LVEF Left ventricular ejection fraction
MIBG Iodine-131-meta-iodobenzylguanidine
MTD Maximum tolerated dose
ORR Overall response rate
OS Overall survival
PD Pharmacodynamic
PFS Progression free survival
PK Pharmacokinetics
PR Partial response
PSD Particle Size Distribution
RANO Response Assessment in Neuro-Oncology
RECIST Response Evaluation Criteria in Solid Tumors
RH Relative Humidity
RP2D Recommended phase II dose
RT Room Temperature
SF Shortening fraction
SGPT Serum glutamic pyruvic transaminase
SGOT Serum glutamic oxaloacetic transaminase
SOA Sucrose Octaacetate
TBI Traumatic brain injury
TOTEM Topotecan-Temozolomide
ULN Upper limit of normal
WBC White blood cell
XRT X-ray therapy

### Example 1: Palatability Assessment of Compound 1B

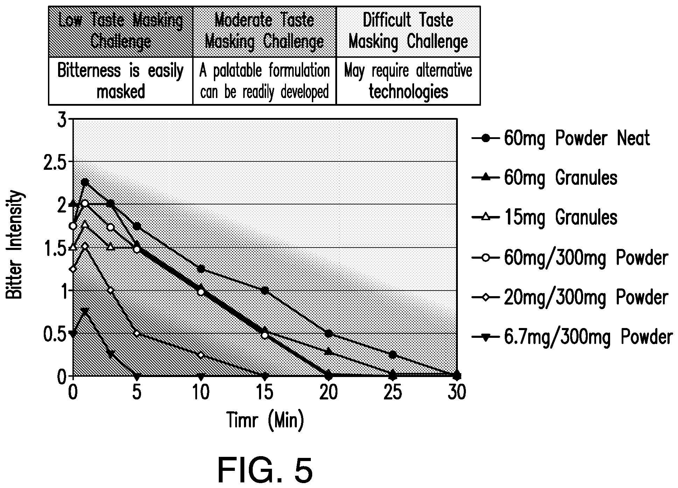

A preliminary taste assessment was conducted in a study where Compound 1B and representative formulations were presented to trained normal health volunteer (NHV) panelists in swish-and-expectorate. The results are summarized in Figure 5. In Figure 5, bitterness intensity is represented on the y-axis, while time from initial tasting is represented on the x-axis. A value of ≤1 represents low taste-masking challenge, while a value ≥ 2 represents difficult taste masking challenge. It was found that Compound 1B had dose-dependent bitterness. At 60 mg, Compound 1B presented a moderate taste masking challenge, but it became a low taste masking challenge at 20 mg. Below 7 mg, the bitterness of the Compound 1B was no longer perceptible.

In addition, it was found that the current IDHIFA^{®} adult formulation did not improve the palatability of Compound 1B at the desired dose. The composition of IDHIFA^{®} formulation is provided in Table B:

**Table B: IDHIFA^{®} formulation**

| Component | Composition (% w/w) | Amount per tablet (mg) | |
|---|---|---|---|
| | | 50 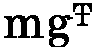 | 100 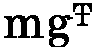 |
| Intragranular | | | |
| Compound 1B | 25 | 60 | 120 |
| Microcrystalline cellulose (Avicel PH-102) | 39.5 | 94.8 | 189.6 |
| Hydroxypropyl cellulose (Klucel EXF) | 2 | 4.8 | 9.6 |
| Sodium starch glycolate | 6 | 14.4 | 28.8 |
| Sodium lauryl sulfate | 1 | 2.4 | 4.8 |
| Hypromellose acetate succinate MF | 1 | 2.4 | 4.8 |
| Colloidal silicon dioxide | 1.5 | 3.6 | 7.2 |
| Magnesium stearate | 0.75 | 1.8 | 3.6 |

| Extragranular | | | |
|---|---|---|---|
| Microcrystalline cellulose (Avicel PH-102) | 20 | 48 | 96 |
| Sodium starch glycolate | 2 | 4.8 | 9.6 |
| Colloidal silicon dioxide | 0.5 | 1.2 | 2.4 |
| Magnesium stearate | 0.75 | 1.8 | 3.6 |
| Total (core, theoretical) | **100.0** | **240** | **480** |
| Opadry II Yellow (85F92450) | 4 | 9.6 | 19.2 |
| Purified water | - * | - * | - * |
| TOTAL (coated, theoretical) | | **249.6** | **499.2** |

| | | | |
|---|---|---|---|
| 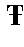 Based on free base (1 mg Compound 1B is equivalent to 0.8312 mg Compound 1C) *Removed during drying of coated tablets | | | |

It was decided that a sweetener is likely required to overcome the bitterness of Compound 1B.

### Example 2: Excipient Selection

Different types of excipients were included into the age-appropriate dosage form. Various sweeteners were screened for their effect on the formulation.

In addition to chemical compatiblity, the selection of the sweet diluent(s) and high-intensity sweetener was also considered for all potential other side effects such as laxative effect, hereditary fructose intolerance, phenylketonuria, diabetes mellitus, and tooth decay.

The chemical compatibility between these excipients and Compound 1B was evaluated through an accelerated stability study. The formulation samples were prepared as blends, then stored at 25°C/60% RH and 40°C/75% RH for up to four weeks.

**Table 1**

| Formulation No. | 1 | 2 | 3 | 4 | 5 | 6 | 7 | 8 | 9 | 10 |
|---|---|---|---|---|---|---|---|---|---|---|
| Component | Concentration (%w/w) | | | | | | | | | |
| | | | | | | | | | | |
| Cpmpound 1B | 25 | 23.575 | 21.175 | 21.175 | 21.175 | 12.5 | 13 | 13 | 13 | 13 |
| Microcrystalline Cellulose PH102 | 59.5 | 56.1085 | 50.3965 | 50.3965 | 50.3965 | 29.75 | 30 | 30 | 30 | 30 |
| Hydroxypropyl Cellulose EXF | 2 | 1.886 | 1.694 | 1.694 | 1.694 | 1 | 1 | 1 | 1 | 1 |
| Sodium Starch Glycolate | 8 | 7.544 | 6.776 | 6.776 | 6.776 | 4 | 4 | 4 | 4 | 4 |
| Sodium Lauryl Sulfate | 1 | 0.943 | 0.847 | 0.847 | 0.847 | 0.5 | 0.5 | 0.5 | 0.5 | 0.5 |
| Hypromellose Acetate Succinate | 1 | 0.943 | 0.847 | 0.847 | 0.847 | 0.5 | 0.5 | 0.5 | 0.5 | 0.5 |
| Colloidal Silicon Dioxide | 2 | 1.886 | 1.694 | 1.694 | 1.694 | 1 | 1 | 1 | 1 | 1 |
| Magnesium stearate | 1.5 | 1.4145 | 1.2705 | 1.2705 | 1.2705 | 0.75 | 0.75 | 0.75 | 0.75 | 0.75 |
| Mannitol | | | | | | 50 | 47 | 41 | 41 | 41 |
| Sucrose | | | | | | | | | | |
| Dextrose | | | | | | | | | | |
| Sucralose | | 5.7 | | | | | 3 | | | |
| Aspartame | | | 15.3 | | | | | 9 | | |
| Acesulfame K | | | | 15.3 | | | | | 9 | |
| Sodium saccharin dihydrate | | | | | 15.3 | | | | | 9 |
| **TOTAL** | **100** | **100** | **100** | **100** | **100** | **100** | **100** | **100** | **100** | **100** |

| Formulation No. | 11 | 12 | 13 | 14 | 15 | 16 | 17 | 18 | 19 | 20 |
|---|---|---|---|---|---|---|---|---|---|---|
| Component | Concentration (%w/w) | | | | | | | | | |
| Cpmpound 1B | 13 | 13 | 13 | 13 | 13 | 13 | 13 | 13 | 13 | 13 |

| Formulation No. | 11 | 12 | 13 | 14 | 15 | 16 | 17 | 18 | 19 | 20 |
|---|---|---|---|---|---|---|---|---|---|---|
| Component | Concentration (%w/w) | | | | | | | | | |
| Microcrystalline Cellulose PH102 | 30 | 30 | 30 | 30 | 30 | 30 | 30 | 30 | 30 | 30 |
| Hydroxypropyl Cellulose EXF | 1 | 1 | 1 | 1 | 1 | 1 | 1 | 1 | 1 | 1 |
| Sodium Starch Glycolate | 4 | 4 | 4 | 4 | 4 | 4 | 4 | 4 | 4 | 4 |
| Sodium Lauryl Sulfate | 0.5 | 0.5 | 0.5 | 0.5 | 0.5 | 0.5 | 0.5 | 0.5 | 0.5 | 0.5 |
| Hypromellose Acetate Succinate | 0.5 | 0.5 | 0.5 | 0.5 | 0.5 | 0.5 | 0.5 | 0.5 | 0.5 | 0.5 |
| Colloidal Silicon Dioxide | 1 | 1 | 1 | 1 | 1 | 1 | 1 | 1 | 1 | 1 |
| Magnesium stearate | 0.75 | 0.75 | 0.75 | 0.75 | 0.75 | 0.75 | 0.75 | 0.75 | 0.75 | 0.75 |
| Mannitol | | | | | | | | | | |
| Sucrose | 50 | 47 | 41 | 41 | 41 | | | | | |
| Dextrose | | | | | | 50 | 47 | 41 | 41 | 41 |
| Sucralose | | 3 | | | | | 3 | | | |
| Aspartame | | | 9 | | | | | 9 | | |
| Acesulfame K | | | | 9 | | | | | 9 | |
| Sodium saccharin dihydrate | | | | | 9 | | | | | 9 |
| **TOTAL** | **100** | **100** | **100** | **100** | **100** | **100** | **100** | **100** | **100** | **100** |

Chemical stability of the blends was evaluated both in the presence and absence of high-intensity sweeteners, and the results are summarized in Table 2.

**Table 2. Assay Potency Results from Formulation Samples to Evaluate Compatibility of Sweeteners with Compound 1B**

| **Formulation No.** | **Sweet Diluent** | **High-intensity Sweetener** | **%Label claim** | | | | |
|---|---|---|---|---|---|---|---|
| | | | **Initial** | **2 weeks¹** | **3 weeks²** | **4 weeks¹** | **4 weeks²** |
| 1 | NA | NA | 98.7 | 97.4 | 97.1 | 96.5 | 97.9 |
| 2 | NA | Sucralose | 99.5 | 98.1 | 99.0 | 101.3 | 98.5 |
| 3 | NA | Aspartame | 100.3 | 97.4 | 98.8 | 98.4 | 97.1 |
| 4 | NA | Acesulfame K | 101.7 | 98.7 | 96.0 | 101.8 | 100.7 |
| 5 | NA | Saccharin | 100.1 | 92.3 | 92.1 | 102.9 | 95.6 |
| 6 | Mannitol | NA | 98.2 | 96.3 | 96.2 | 96.6 | 95.1 |
| 7 | Mannitol | Sucralose | 97.7 | 99.0 | 98.3 | 98.7 | 98.5 |
| 8 | Mannitol | Aspartame | 98.4 | 96.6 | 94.3 | 95.0 | 95.8 |
| 9 | Mannitol | Acesulfame K | 98.6 | 98.5 | 93.9 | 93.7 | 102.1 |
| 10 | Mannitol | Saccharin | 93.6 | 95.7 | 93.0 | 99.6 | 99.2 |
| 11 | Sucrose | NA | 94.6 | 75.9 | 91.3 | 84.8 | 108.9 |
| 12 | Sucrose | Sucralose | 96.1 | 101.3 | 88.0 | 95.8 | 95.9 |
| 13 | Sucrose | Aspartame | 98.4 | 96.0 | 113.9 | 95.4 | 93.6 |
| 14 | Sucrose | Acesulfame K | 94.1 | 90.4 | 84.0 | 90.8 | 86.1 |
| 15 | Sucrose | Saccharin | 80.6 | 85.4 | 84.3 | 73.2 | 73.5 |
| 16 | Dextrose | NA | 97.6 | 94.5 | 91.5 | 89.2 | 92.0 |
| 17 | Dextrose | Sucralose | 100.2 | 103.4 | 99.6 | 101.3 | 93.4 |
| 18 | Dextrose | Aspartame | 97.8 | 93.6 | 93.3 | 105.5 | 98.1 |
| 19 | Dextrose | Acesulfame K | 94.5 | 91.6 | 95.8 | 96.6 | 92.6 |
| 20 | Dextrose | Saccharin | 95.8 | 96.4 | 96.6 | 97.1 | 89.8 |

| | | | | | | | |
|---|---|---|---|---|---|---|---|
| ¹40 °C/75%RH ²25 °C/60%RH at 2 weeks for samples containing dextrose, 3 weeks for the other samples | | | | | | | |

The results showed that in general, no reduction in assay potency values was observed for samples containing mannitol, whereas samples containing dextrose and sucrose showed decreasing potency values. For the dextrose-containing samples, the color turned yellowish following storage at 40 °C/75% RH, although no reduction in potency values was intially observed. Thus, the preferred sweet diluent to be included in age-appropriate Compound 1B pdiatric formulation was mannitol. It was also found that samples containing the high-intensity sweetener saccharin showed a reduction in potency values, but the other high-intensity sweeteners evaluated were chemically compatible with Compound 1B.

A palatability study was conducted using high intensity sweeteners in a mimetic system containing 0.0275% sucrose octaacetate (SOA) that mimicked the bitter profile of Compound 1B. The results showed that acesulfame K provided minimal bitterness reduction, whereas sucralose was effective at reducing bitterness. The effects of substituting mannitol with sucrose or dextrose, varying the levels of sucralose, as well as reduction and elimination of mannitol in the presence of sucralose to the palatability of the mimetic system were also explored. The results showed that both mannitol and sucralose were needed to minimize the bitter taste of Compound 1B, and any reduction in the proportion of mannitol resulted in a higher bitterness intensity. In addition, an optimum level of sucralose at 4.4% was found to provide an appropriate flavor balance in the mannitol-sucralose sweetener system.

When taking into consideration the chemical incompatibility between saccharin and Compound 1B and the potential PKU liability of aspartame, the mannitol-sucralose sweetener system again emerged as the most appropriate system for the Compound 1B pediatric formulation, as summarized in Table 3.

**Table 3. Selection of the Sweetener System for Compound 1B Pediatric Age-Appropriate Dosage Form**

| **Sweetener** | **Chemical stability** | **Improved palatability** | **Comments** |
|---|---|---|---|
| Sucralose | Compatible | Yes | Commonly used as artificial sweetener |
| Acesulfame-K | Compatible | No | |
| Saccharin | Incompatible | No | |
| Aspartame | Compatible | Not tested | Not tested for palatability due to PKU concern |
| Sucralose + sucrose | Incompatible | Yes | Sucrose samples showed discoloration |
| Sucralose + dextrose | Inconclusive | Yes | Dextrose samples showed discoloration |
| Sucralose + mannitol | Compatible | Yes | The best choice - mannitol is commonly used in dry granulation and known to improve mouthfeel |

### Example 3: Process and Packaging Risk Assessment

The minitablets were packaged in capsules, which were subsequently placed in HDPE bottles with desiccants canister. The choice of minitablets maximizes dose recovery and minimizes potential accidental exposure of caregivers, while selected packaging provides better protection against moisture compared to packaging in sachets.

Due to the small weight of each minitablet, a multi-tip tooling is needed during compression, since it would reduce the sensitivity of the tooling to variations in compression force caused by variability in fill weight by dispersing the force per tooling over a much larger effective area. In addition, the use of multi-tip tooling would increase the production throughput.

The compression of minitablets added one additional unit operation compared to dry granulation. However, the selection of minitablets would present a reduced risk of exposure for caregivers when compared against oral granules. In addition, the dose recovery from a minitablet dosage form was expected to be higher when compared against oral granules, since the adherence to the capsule shell was expected to be much lower. In order to evaluate this, a study was performed comparing the quantities of minitablets and oral granules recovered after filling into capsules. When compared against the original quantities, it was found that the proportion of minitablets recovered from the capsules were indeed higher than the proportion of oral granules recovered, *see* Table 4. Although the difference between the two was relatively small (99.95% vs. 99.08%), this difference was statistically significant.

**Table 4: Recovery from minitablets and oral granules**

| | **Minitablets** | | | **Oral granules** | | |
|---|---|---|---|---|---|---|
| **Sample #** | **Weight of fill material, initial (mg)** | **Weight of fill material, recovered (mg)** | **% dose recovered** | **Weight of fill material, initial (mg)** | **Weight of fill material, recovered (mg)** | **% dose recovered** |
| 1 | 263 | 262 | 99.62 | 249 | 246 | 98.80 |
| 2 | 253 | 253 | 100.00 | 251 | 249 | 99.20 |
| 3 | 253 | 253 | 100.00 | 251 | 246 | 98.01 |
| 4 | 255 | 255 | 100.00 | 253 | 251 | 99.21 |
| 5 | 254 | 254 | 100.00 | 256 | 252 | 98.44 |
| 6 | 253 | 254 | 100.40 | 256 | 253 | 98.83 |
| 7 | 252 | 252 | 100.00 | 252 | 250 | 99.21 |
| 8 | 251 | 251 | 100.00 | 250 | 249 | 99.60 |
| 9 | 257 | 257 | 100.00 | 253 | 251 | 99.21 |
| 10 | 253 | 253 | 100.00 | 251 | 248 | 98.80 |
| 11 | 255 | 255 | 100.00 | 252 | 251 | 99.60 |
| 12 | 253 | 253 | 100.00 | 254 | 252 | 99.21 |
| 13 | 257 | 256 | 99.61 | 254 | 253 | 99.61 |
| 14 | 257 | 256 | 99.61 | 250 | 248 | 99.20 |
| 15 | 250 | 250 | 100.00 | 256 | 254 | 99.22 |
| Average | 254.4 | 254.3 | 99.95 | 252.5 | 250.2 | 99.08 |
| Stdev | 3.2 | 2.9 | 0.20 | 2.3 | 2.5 | 0.44 |
| %RSD | 1.3 | 1.1 | 0.20 | 0.9 | 1.0 | 0.44 |

### Example 4: Lead Product Presentation and Manufacturing Process

As an outcome of the risk assessment exercises and feasibility experiments, minitablets for sprinkle onto soft food was selected as the lead product presentation for the age-appropriate Compound 1B pediatric dosage form. The development of this dosage form was initiated with the adult formulation, although it was recognized that the drug load may need to be lowered from 25% to 10-15%. The weight of each minitablet was designed to be approximately 1.67 mg, with a diameter and height of 1.2 mm. The selection of the size and compression weight of each minitablet was done for two reasons: first, the small size of the minitablet would help with ease of swallowing during administration. Second, with the compression weight selected, the number of minitablets required to deliver 20 mg enasidenib is relatively large (estimated as 100-200 minitablets depending on the final concentration), increasing the probability that each capsule/sachet would contain an accurate dose, since small variations in the number of minitablets from capsule to capsule or sachet to sachet would not result in meaningful dose variability.

With relatively low compression weight and small minitablet diameter, it was important that the final blend formulation had good flow properties. Thus, the dry granulation manufacturing method was preferred compared to direct blend, which matches the process currently used to manufacture IDHIFA^{®} adult tablets. Compression was performed using multi-tip tooling, as was highlighted following the risk assessment exercise.

For packaging, minitablets filled into capsules was selected as the primary option, because it allows a high degree of protection against moisture. When the minitablets are filled into hypromellose-based capsules, the capsules can be filled into HDPE bottles, where desiccants can be added to provide extra protection. The bottles can subsequently be placed inside a foil-lined pouch and heat-sealed for even further protection. This level of protection against moisture is more comprehensive than protection from packaging in sachets. A preliminary assessment of the physical stability of Compound 1B as minitablets packaged in sachets showed rapid conversion to the amorphous form, *i.e.,* Compound 1D, even when packaging was done at RH<30% RH, and maintained at or below 30% RH.

In a separate experiment, %amorphous content, *i.e.,* Compound 1D, was assessed when the minitablets were stored in sachets. The content of Compound 1D was 30.1% after 6 months storage at 40 °C/75% RH, whereas the same formulation contained 11.3% Compound 1D when stored in HDPE bottles with 2 g desiccants. This result suggested that the protection afforded through packaging in sachets was not sufficient to prevent conversion of Compound 1B into Compound 1D.

### Example 5: Minitablet Manufacturing Process and Formulation Composition Development

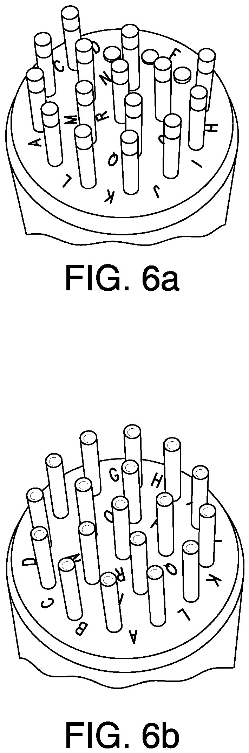

Once minitablets-in-capsule product presentation was selected, the manufacturing process and formulation composition development was initiated. Roller compaction was selected as the manufacturing process to ensure good final blend flow properties. A batch was first prepared according to IDHIFA^{®} adult tablet composition as described in US Patent Application Publication No. 2018/0064715A1, roller compacted into granules, then compressed into 1.2 mm minitablets. However, sticking problem was immediately observed during compression as seen in Figure 6a.

Additional batches were prepared with lowered drug loads (12.5 and 6.25%) as well as incorporation of dextrose or mannitol (*see* formulations A-7 and A-8 in Table 5). As the drug load was decreased, the sticking tendency was reduced, but filming was observed even at 6.25% drug load. The disintegration time of the minitablets was very rapid (less than 10 seconds, *see* formulation A-7), even at 0.95 solid fraction value.

A formulation was then designed with 9.38% Compound 1B, and the proportions of the intra- and extra-granular lubricant (Mg-stearate) was adjusted. Sucralose was included in the formulation to offer better palatability, and three different levels of mannitol (0, 25, and 35%) were evaluated (*see* formulation A-11 in Table 5). This formulation no longer exhibited sticking or filming tendency when compressed into minitablets (see Figure 6b). The bulk density values were 0.494, 0.495, and 0.527 g/mL for formulations containing 0, 20, and 35% mannitol, respectively, showing an increasing trend as the proportion of mannitol was increased and the MCC decreased. Sieve analysis of the milled granules showed comparable particle size distribution for the two formulations containing 25 and 35% mannitol, while the formulation containing 0% mannitol had more particles retained on 150 micron sieve opening size and less particles retained on 500 and 355 micron sieve openings.

This formulation containing intra- and extra-granular lubricant and mannitol was further optimized into the final recommended formulation (see Table 10), which contains 9.62% Compound 1B (8.0% as free base). A capsule containing 250 mg minitablets (approximately 150 minitablets by count) would deliver the intended dose of 20 mg Compound 1C.

**Table 5. Unit Formula of Compound 1B Pediatric Minitablet Formulation Prototypes**

| **Component** | **Amount per tablet (mg)** | | | | | | | | |
|---|---|---|---|---|---|---|---|---|---|
| | A-7 | | A-8 | A-10 | A-11 | | | A-12 | A-13 |
| | 12.5% DL, mannitol | 12.5% DL, dextrose | 6.25% DL, MCC only | 1.2 mm, 6.25% DL, RC, w/ mannitol | 1.2 mm, 9.38% DL, 0% mannitol | 1.2 mm, 9.38% FB DL, 20% mannitol | 1.2 mm, 9.38% FB DL, 35% mannitol | 1.2 mm, 9.62% DL | 1.2 mm, 9.62% DL, conf. stability |
| Intragranular | | | | | | | | | |
| Compound 1B | 24.1 | 24.1 | 24.1 | 24.1 | 24.1 | 24.1 | 24.1 | 24.1 | 24.1 |
| Microcrystalline cellulose (Avicel PH-102) | 38.0 | 38.0 | 224.3 | 19.2 | 119.7 | 68.3 | 29.8 | 48.2 | 48.1 |
| Hydroxypropyl cellulose (Klucel EXF) | 1.9 | 1.9 | 7.7 | 7.7 | 5.1 | 5.1 | 5.1 | 5.0 | 5.0 |
| Sodium starch glycolate | 5.8 | 5.8 | 23.1 | 23.1 | 15.4 | 15.4 | 15.4 | 15.0 | 15.0 |
| Sodium lauryl sulfate | 1.0 | 1.0 | 3.8 | 3.8 | 2.6 | 2.6 | 2.6 | 2.5 | 2.5 |
| Hypromellose acetate succinate MF | 1.0 | 1.0 | 3.8 | 3.8 | 2.6 | 2.6 | 2.6 | 2.5 | 2.5 |
| Colloidal silicon dioxide | 1.4 | 1.4 | 5.8 | 5.8 | 3.8 | 3.8 | 3.8 | 3.8 | 3.8 |
| Mannitol | 96.2 | - - | - - | 177.1 | - - | 51.3 | 89.8 | 68.3 | 68.1 |
| Dextrose | - - | 96.2 | - - | - - | - - | - - | - - | - - | - - |
| Sucralose | - - | - | - - | 5.8 | 11.5 | 11.5 | 11.5 | 11.0 | 11.0 |
| Magnesium stearate | 0.7 | 0.7 | 2.4 | 2.9 | 1.3 | 1.3 | 1.3 | 1.3 | 1.3 |

| Extragranular | | | | | | | | | |
|---|---|---|---|---|---|---|---|---|---|
| Microcrystalline cellulose (Avicel PH-102) | 19.2 | 19.2 | 64 | 99.1 | 61.6 | 61.6 | 61.6 | 60.2 | 60.0 |
| Sodium starch glycolate | 1.9 | 1.9 | 6.4 | 7.7 | 5.1 | 5.1 | 5.1 | 5.0 | 5.0 |

| **Component** | **Amount per tablet (mg)** | | | | | | | | |
|---|---|---|---|---|---|---|---|---|---|
| | A-7 | | A-8 | A-10 | A-11 | | | A-12 | A-13 |
| | 12.5% DL, mannitol | 12.5% DL, dextrose | 6.25% DL, MCC only | 1.2 mm, 6.25% DL, RC, w/ mannitol | 1.2 mm, 9.38% DL, 0% mannitol | 1.2 mm, 9.38% FB DL, 20% mannitol | 1.2 mm, 9.38% FB DL, 35% mannitol | 1.2 mm, 9.62% DL | 1.2 mm, 9.62% DL, conf. stability |
| Colloidal silicon dioxide | 0.5 | 0.5 | 1.6 | 1.9 | 1.3 | 1.3 | 1.3 | 1.3 | 1.3 |
| Magnesium stearate | 0.7 | 0.7 | 2.4 | 2.9 | 2.6 | 2.6 | 2.6 | 2.5 | 2.5 |
| **TOTAL** | **192.5** | **160.0** | **320.0** | **385.0** | **256.7** | **256.7** | **256.7** | **250.6** | **250.0** |

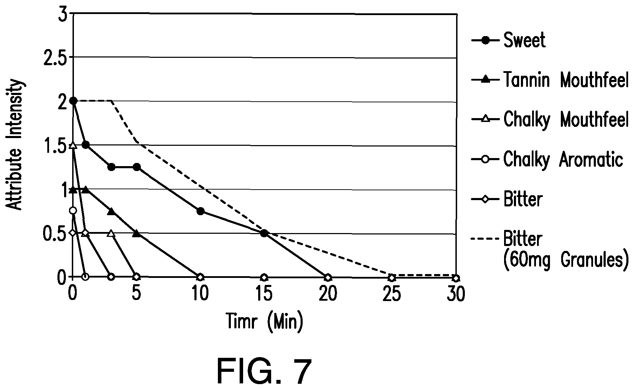

To ensure that the new formulation would have acceptable palatability, a taste assessment evaluation was conducted with the formulation described in Table 10. The results, presented in Figure 7, showed that the formulation containing sucralose and mannitol as sweeteners reduced the bitterness of Compound 1B to a very slight intensity, below the level of patient perception (1.0). This result is a significant improvement when compared against granules prepared according to the adult formulation.

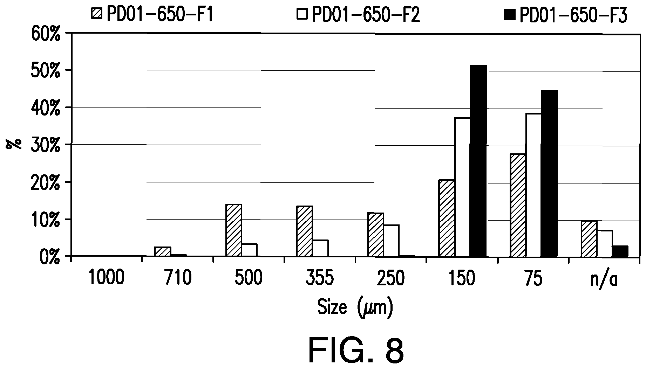

Formulation A-12 described in Table 5 was prepared through dry granulation manufacturing method at three different roll pressures: 0, 2, and 4 kN/cm designated as F3, F2 and F1, respectively. The particle size distribution and bulk/tapped densities of milled granules prepared from ribbons produced at different roll pressures are presented in Figure 8 and Table 6. The size of particles produced following roller compaction at 0 kN/cm (direct compression) was smaller than the other two conditions, and showed primarily the nominal particle size of the starting excipients (100-200 µm).

**Table 6. Bulk Density, Tapped Density, and Hausner Ratio of Blends Prepared**

| **Sample** | **Bulk density (g/mL)** | **Tapped density* (g/mL)** | **Hausner ratio** |
|---|---|---|---|
| Intra-granular blend, F1 (4 kN/cm) | 0.56 | 0.744 | 1.33 |
| Intra-granular blend, F2 (2 kN/cm) | 0.51 | 0.726 | 1.42 |
| Intra-granular blend, F3 (0 kN/cm) | 0.40 | 0.632 | 1.58 |
| Final blend, F2 | 0.49 | 0.700 | 1.43 |

| | | | |
|---|---|---|---|
| *1000 taps | | | |

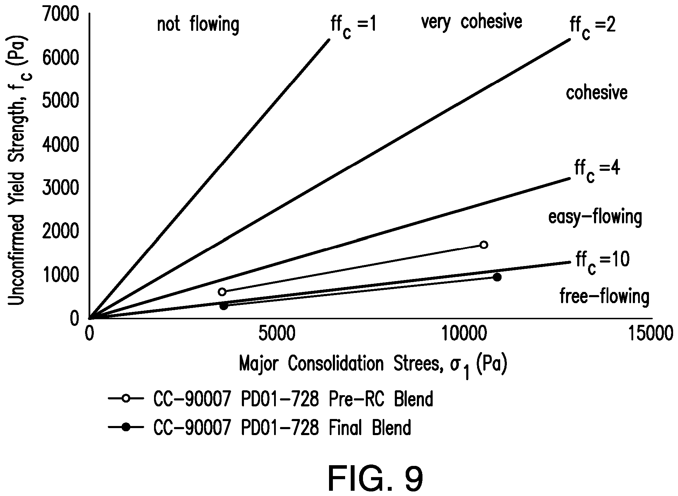

The flow property of the final blend was assessed using shear cell analysis, and the result, presented in Figure 9, showed that the flow property of the blend improved from easy flowing prior to roller compaction to free flowing after granulation and mixing with extra granular material.

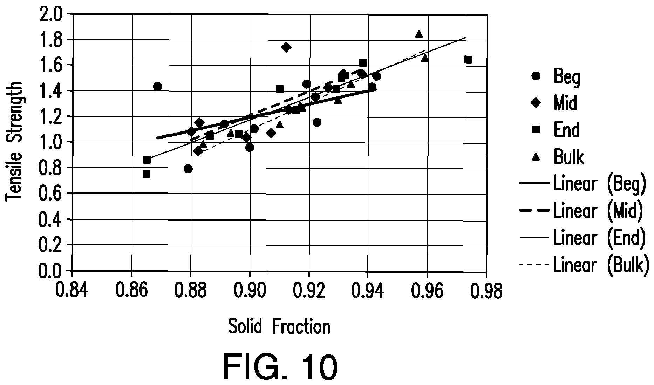

The blend was compressed into minitablets, and the results, presented in Figure 10, showed that minitablets with 0.9 to 0.95 target solid fraction would have a tensile strength of 1.0 to 1.6 MPa, which was deemed sufficiently high to allow robust handling during encapsulation and packaging.

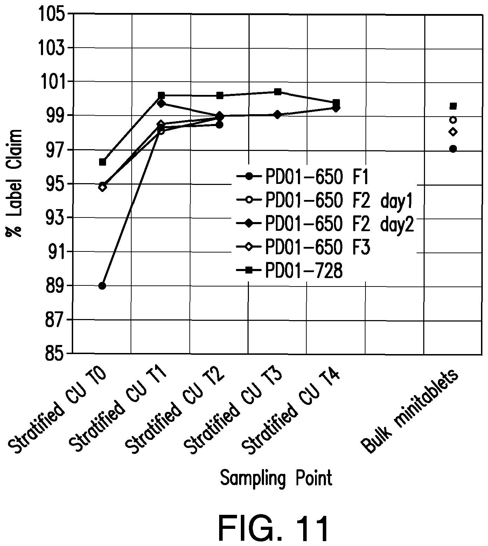

During compression of formulations A-12 and A-13, stratified content uniformity (CU) samples were pulled and analyzed for % assay. The results provided in Figure 11 demonstrated that the potency values from the first samples pulled were 89-96% of target value. However, as the compression progressed, the potency value increased to ~100%. These results suggested that the final blend might have had a tendency for segregation during tablet press charging. Since the potency of minitablets increased to 100%, the lower potency value at the beginning of compression process could be mitigated by discarding the initial samples.

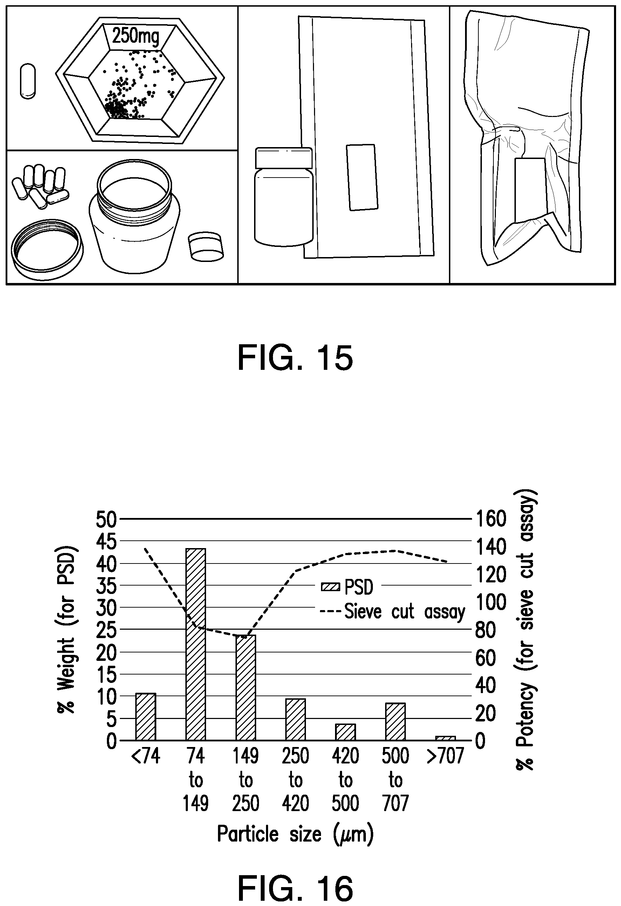

Figure 16 provides particle size distribution and sieve cut assay analysis for samples from the middle roller compaction force condition (2.5 kN/cm) for the final blend.

The minitablets having formulation A-13 were manually encapsulated and packaged into bottles for stability study.

### Example 6: Performance Against Adult Dosage Form (In-Vitro Dissolution)

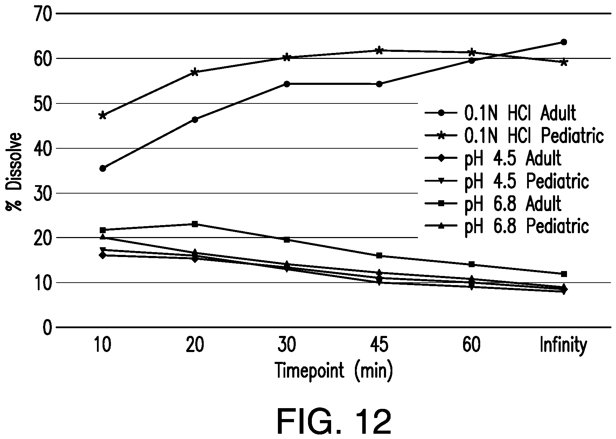

The drug release performance of Compound 1B pediatric minitablets was assessed by measuring the dissolution profiles of Compound 1B from the minitablets packaged in five capsules and comparing the results against the dissolution profiles of enasidenib from 100 mg IDHIFA^{®} adult tablets (N=12). Three different media representing physiologically relevant pHs were used: 0.1 N HCl, pH 4.5 acetate buffer, and pH 6.8 phosphate buffer. The results provided in Figure 12 showed that Compound 1B release profiles from the minitablets at all pHs measured were similar to the release profiles from 100 mg IDHIFA^{®} adult tablets, with F2 values of 55, 95, and 68 for the three media, respectively.

### Example 7: Stability Confirmation Data

### A. Dose Recovery

An experiment was conducted to compare the dose recovered for minitablets packaged in capsules and sachets. The results showed that the recovery of minitablets packaged in sachets was slightly higher than the minitablets or oral granules packaged in capsules. However, the recovered weight from both options evaluated were greater than 95% of the target weight, and it was concluded that primary packaging in both capsules and sachets will allow acceptable recovery of the dose upon administration. The composition used in this study was the same as that provided in Table B.

### B. Chemical Stability

A stability study of a representative Compound 1B pediatric formulation in the proposed packaging was conducted. The formulation having the composition in Table 10 was used in this study. The results, presented in Table 7, showed that no meaningful difference in assay, degradation products, or dissolution rate was observed after six months storage at 5 °C, 25 °C/60% RH, and 40 °C/75% RH.

**Table 7. The Most Current Results from Stability Study of Minitablets-in-Capsules**

| Storage condition | | 5 °C | | | | 25 °C/60% RH | | | |
|---|---|---|---|---|---|---|---|---|---|
| Test name | | Initial | 1 Month | 3 Months | 6 Months | Initial | 1 Month | 3 Months | 6 Months |
| Appearance | | Conforms | Conforms | Conforms | Conforms | Conform s | Conform s | Conforms | Conforms |
| Assay | | 99.3 | 99.8 | 99.7 | 99.9 | 99.3 | 99.5 | 100.3 | 100.4 |
| Degradation products | 1-((4-amino-6-(6-(trifluoromethyl)pyridi n-2-yl)-1,3,5-triazin-2-yl)amino)-2-methylpropan-2-ol | ND | ND | ND | ND | ND | ND | ND | ND |
| | 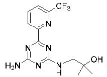 | | | | | | | | |
| | 4-[(2-hydroxy-2-methylpropyl)amino]-6-[6-(trifluoromethyl)pyridi n-2-yl]-1,3,5-triazin-2-ol | ND | ND | ND | ND | ND | ND | ND | ND |
| | 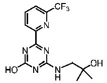 | | | | | | | | |
| Dissolution | 10 minutes | 59 | 60 | 67 | 67 | 59 | 59 | 68 | 68 |
| | 15 minutes | 75 | 77 | 74 | 74 | 75 | 76 | 75 | 75 |
| | 20 minutes | 79 | 82 | 80 | 80 | 79 | 82 | 81 | 80 |
| | 30 minutes | 86 | 89 | 86 | 86 | 86 | 87 | 87 | 86 |
| | 45 minutes | 92 | 94 | 91 | 91 | 92 | 93 | 92 | 91 |
| | 60 minutes | 94 | 96 | 94 | 93 | 94 | 95 | 95 | 92 |
| | 90 minutes | 97 | 99 | 97 | 96 | 97 | 98 | 98 | 96 |
| Storage condition | 5 °C | | | | 25 °C/60% RH | | | | |
| Test name | Initial | 1 Month | 3 Months | 6 Months | Initial | 1 Month | 3 Months | 6 Months | |
| Water activity | 0.09 | 0.07 | 0.07 | 0.08 | 0.09 | 0.1 | 0.1 | 0.09 | |
| Water content (average) | 1.5% | 1.4% | 1.1% | 1.2% | 1.5% | 1.4% | 1.0% | 1.1% | |

| | | | | | |
|---|---|---|---|---|---|
| Storage condition | | 40 °C/75% RH | | | |
| Test name | | Initial | 1 Month | 3 Months | 6 Months |
| Appearance | | Conforms | Conforms | Conforms | Conforms |
| Assay | | 99.3 | 99.5 | 99.9 | 100.1 |
| Degradation products | 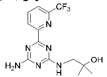 1-((4-amino-6-(6-(trifluoromethyl)pyridin-2-yl)-1,3,5-triazin-2-yl)amino)-2-methylpropan-2-ol | ND | ND | ND | ND |
| | 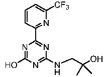 4-[(2-hydroxy-2-methylpropyl)amino]-6-[6-(trifluoromethyl)pyridin-2-yl]-1,3,5-triazin-2-ol | ND | ND | ND | ND |
| Dissolution | 10 minutes | 59 | 62 | 70 | 69 |
| | 15 minutes | 75 | 77 | 77 | 76 |
| | 20 minutes | 79 | 82 | 82 | 81 |
| | 30 minutes | 86 | 88 | 88 | 87 |
| | 45 minutes | 92 | 93 | 92 | 91 |
| | 60 minutes | 94 | 95 | 95 | 93 |
| | 90 minutes | 97 | 98 | 97 | 96 |
| Water activity | | 0.09 | 0.11 | 0.11 | 0.1 |
| Water content (average) | | 1.5% | 1.4% | 1.1% | 1.1% |

### C. Physical Stability

Physical stability was an important consideration during the development of Compound 1B pediatric formulation, since it was found that exposure of Compound 1B to high relative humidity led to the formation of Compound 1D. During the development of IDHIFA^{®} adult tablets, the formation of Compound 1D was minimized through engineering controls (i.e. by limiting the RH that Compound 1B and the tablets were exposed to during manufacturing and storage). Due to changes to the formulation composition of age-appropriate Compound 1B pediatric formulation, the sensitivity of the formulation to moisture could have changed when compare against IDHIFA^{®} adult tablets.

To evaluate the potential impact of moisture to the physical stability of Compound 1B in the pediatric formulation, powder blends and minitablets prepared according to the formulation specified in Table 10 was stored in open dishes at 30 °C at increasing relative humidity from 0 to 50% RH in 10% RH increment. Periodically, % of Compound 1D in the samples was quantified via ssNMR and compared against samples with known quantities of Compound 1D and crystalline Compound 1B. The results, presented in Table 8, showed that the formation of Compound 1D at room temperature was relatively slow below 30% RH, and increased significantly above 30% RH. At high RH, Compound 1D that had formed further converted into crystalline free base Form 1. The data also showed that compression of the blend into minitablets resulted in an increase in the % amorphous content.

**Table 8. Compound 1D Contents of Pediatric Minitablets and Powder Blends Stored on Open Dishes**

| **Sample** | **Storage condition** | **Amorphous content** | |
|---|---|---|---|
| | | **4 weeks** | **8 weeks** |
| Minitablets | 30 °C/0% RH | 8% | 12% |
| | 30 °C/10% RH | 10% | 13% |
| | 30 °C/20% RH | 10% | 14% |
| | 30 °C/30% RH | 14% | N/A, contained form B |
| | 30 °C/40% RH | N/A, contained form B | N/A, contained form B |
| | 30 °C/50% RH | N/A, contained form B | N/A, contained form B |
| Powder blends | 30 °C/0% RH | 3% | 6% |
| | 30 °C/10% RH | 5% | 7% |
| | 30 °C/20% RH | 5% | 7% |
| | 30 °C/30% RH | 6% | 8% |
| | 30 °C/40% RH | N/A, contained form B* | 9%* |
| | 30 °C/50% RH | N/A, contained form B | N/A, contained form B |

| | | | |
|---|---|---|---|
| *trend observed between 4 and 8 weeks may have been caused by sample sub-sampling or analytical variability | | | |

In addition to the open dish study described above, a study was conducted to assess the amount of Compound 1D in encapsulated minitablets when packaged in HDPE bottles with 2 g desiccants included in each bottle, and aluminum foil pouch over packaging The results showed that a step increase was observed between the initial samples and samples pulled after 1-month storage under different conditions. However, Compound 1D content beyond the 1-month time point increased at a very slow rate, with a faster trend for samples stored at higher temperatures and relative humidities.

**Table 9. Compound 1D Contents of Encapsulated Pediatric Minitablets Packaged in Bottles with Desiccants**

| **Sample condition** | **Time Point** | | | | |
|---|---|---|---|---|---|
| | **Initial** | **1-Month** | **3-Month** | **4-Month** | **6-Month** |
| 5 °C | 8% | 11% | 11% | 11% | 12% |
| 25 °C/60% RH | | 10% | 12% | 12% | 13% |
| 40 °C/75% RH | | 11% | 12% | 12% | 14% |

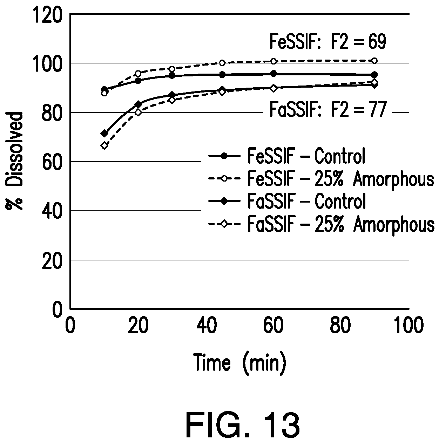

The data also showed that the amount of Compound 1D present in the minitablets was in general higher than what was observed with IDHIFA^{®} adult tablets. To ensure that this change would not result in any impactful difference, the dissolution rates of Compound 1B from tablets containing all 100% crystalline Compound 1B and 75% crystalline Compound 1B /25% Compound 1D were assessed in media at different pH and in biorelevant media, fed state simulating intestinal fluid (FeSSIF) and fasted state simulated intestinal fluid (FaSSIF). The results, presented in Figure 13, showed that the dissolution rates of Compound 1B from the two products were similar.

### Example 8: Food Compatibility

Since Compound 1B pediatric minitablets may be mixed with food to assist the dosing, the compatibility of the minitablets with different foods was assessed. Strawberry Jell-O^{®} low-calorie gelatin snacks, vanilla Jell-O^{®} pudding snacks, and strawberry Dannon^{®} non-fat yogurt (vitamin A and D, light and fit) were used for the assessment based on feedback from the clinical oncology nurses at the study center. The results show that these food products are compatible for co-administration with the three food products mentioned, and no chemical or physical incompatibiity was observed.

### Example 9: Final formulation

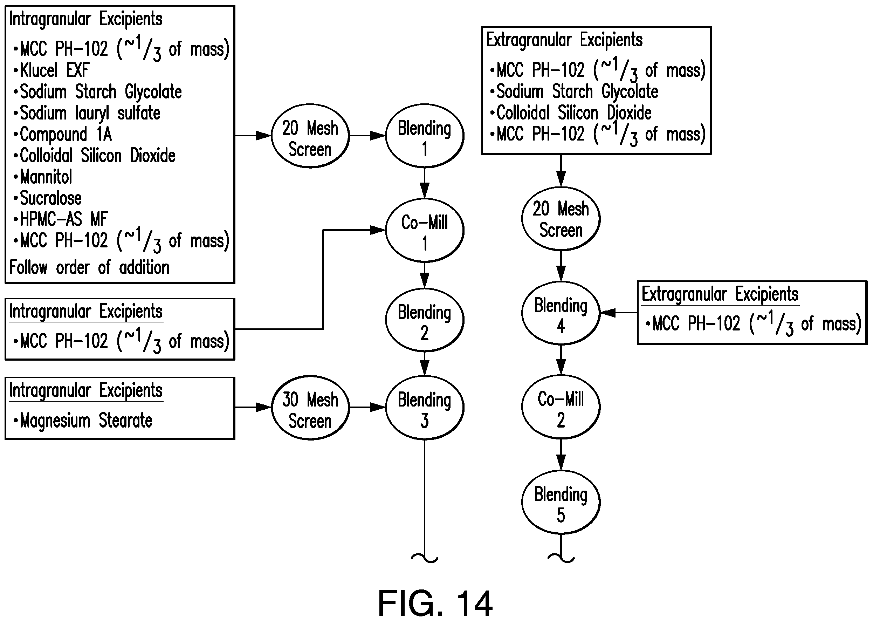

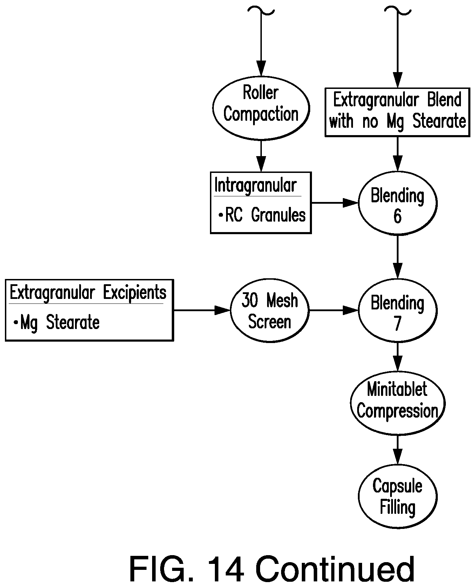

Based on the development efforts, a minitablet formulation was decided as shown in Table 10. The manufacturing process for the formulation is summarized in Figure 14. The compressed minitablets would be encapsulated into size 00 Vcaps Plus Swedish orange hypromellose capsules, which could be opened to sprinkle the minitablets onto soft foods. The capsules would be packaged in HDPE bottles with induction-sealed child-resistant closure. To provide extra protection against moisture, a 2-g desiccant canister would be added inside each bottle, and the bottles would be packaged in aluminum pouches, which would subsequently be heat sealed. To maximize the shelf life of the formulation, the bottles would be stored at refrigerated (2-8 °C) temperature. Figure 15 demonstrartes packaging scheme and product presentation for minitablets-in-capsules. Figure 16 provides particle size distribution and sieve cut assay analysis

**Table 10. Compound 1B Pediatric Formulation Composition**

| **Ingredient** | **Function** | **Amount** | |
|---|---|---|---|
| | | **% w/w** | **Per 1 capsule (250 mg, approximately 150 minitablets)** |
| Compound 1B | API | 9.62 | 24.05* |
| Microcrystalline cellulose (Avicel^{®} PH-102) | Binder/filler | 43.24 | 108.1 |
| Mannitol (Pearlitol^{®} SD200) | Sweet diluent | 27.24 | 68.1 |
| Hydroxypropyl cellulose (Klucel^{™} EXF Pharm) | Binder | 2.0 | 5.0 |
| Sodium starch glycolate (Explotab^{®}) | Disintegrant | 8.0 | 20.0 |
| Sodium lauryl sulfate (Stepanol^{®} WA100) | Wetting agent | 1.0 | 2.5 |
| Colloidal SiO₂ (Cab-O-Sil^{®} M5P) | Flow agent | 2.0 | 5.0 |
| Hypromellose acetate succinate (AS-MF) | Stabilizer | 1.0 | 2.5 |
| Sucralose | High-intensity sweetener | 4.4 | 11.0 |
| Magnesium stearate vegetable (HyQual) | Lubricant | 1.5 | 3.75 |
| **Total core** | | **100** | **250.0** |

| | | | |
|---|---|---|---|
| *equivalent to 20 mg enasidenib as free base | | | |

Figure 16 provides particle size distribution and sieve cut assay analysis

### Example 10: Proof-of-Concept Therapeutic Stratification Trial of Molecular Anomalies in Relapsed or Refractory Tumors

### OBJECTIVES

### Primary Objectives:

1. To evaluate the safety, antitumor activity, and pharmacokinetic of enasidenib in relapsed or refractory acute myeloid leukaemia paediatric patients with isocitrate dehydrogenase 2 (IDH2) mutation from 2 to leass that 18 years of age.

### Secondary Objectives:

To characterize the PK/pharmacodynamic relationships of AG-221 and 2-hydroxygluturate (2HG).
- Signal of activity based on the 2003 revised International Working Group criteria for AML.

### STUDY DESIGN

This is an international, multi-center, single-arm, open-label, prospective, phase II dose-validation study with a RP2D confirmation part, which is open to being expanded to refine an efficacy assessment for each agent.

For all patients, an extensive molecular analysis of their tumor tissue performed prior to study entry will be requested that will serve to select the most appropriate treatment.
If no molecular alteration in the pathways selected for this trial are identified, patients may still be enrolled into this study according to physician discretion, if a strong scientific rationale exists to support the notion that the patient may derive clinical benefit, and if the patient fulfills all other inclusion and no exclusion criteria.

### STUDY POPULATION

1. Age: Patients must be ≥ 24 months and ≤ 18 years of age at the time of study enrollment.
2. Diagnosis: Patients with AML in the 2nd or greater relapse or with refractory AML after ≥ two prior induction attempts.
a. Each block of chemotherapy (i.e. ADE, MA) is a separate re- induction attempt.
b. Donor lymphocyte infusion (DLI) is considered a re-induction attempt.
c. AML associated with Down Syndrome or t(15;17) are not eligible for study

3. Performance Level: Karnofsky ≥ 50% for patients > 16 years of age and Lansky ≥ 50 for patients ≤ 16 years of age.
4. Disease Status: Bone marrow assessment must have at least one of the following:
a. ≥ 5 % blasts by morphology and/or flow cytometry
b. Minimal residual disease by flow cytometry
c. Any molecular evidence of disease
   Patient must have documented IDH2 gene-mutated disease obtained from a peripheral blood or bone marrow sample at the time of diagnosis and/or relapsed/refractory disease. Mutations in IDH2 at R140 or R172 are eligible for enrollment.

5. Therapeutic Options: Patient's current disease state must be one for which there is no known curative therapy or therapy proven to prolong survival with an acceptable quality of life.
6. CNS Status: No intrathecal therapy will be permitted on study. Subjects with known CNS leukemia or has clinical symptoms suggesting active central nervous system (CNS) leukemia will not be eligible. Therefore, only patients with CNS1 disease will be eligible for study. CNS1 status is defined as absence of blasts in cerebral spinal fluid (CSF) on cytospin preparation, regardless of the number of WBCs in the CSF.
7. Prior Therapy: Patients must have fully recovered from the acute toxic effects of all prior anti-cancer therapy to ≤ Grade 2 prior to enrollment and must meet the following minimum duration from prior anti-cancer directed therapy prior to enrollment. If after the required timeframe, the numerical eligibility criteria are met, e.g., blood count criteria, the patient is considered to have recovered adequately.
a. Cytotoxic chemotherapy or other anti-cancer agents known to be myelosuppressive.
   - ≥ 14 days must have elapsed after the completion of other cytotoxic therapy, with the exception of hydroxyurea. Additionally, patients must have fully recovered from all acute toxic effects of prior therapy.
   - Intrathecal chemotherapy must be completed ≥ 72 hours prior to the start of the first cycle of treatment.
   NOTE: Cytoreduction with hydroxyurea must be discontinued ≥ 24 hours prior to the start of protocol therapy.
b. Anti-cancer agents not known to be myelosuppressive (e.g. not associated with reduced platelet or ANC counts): ≥ 7 days after the last dose of agent.
c. Antibodies: ≥ 21 days must have elapsed from infusion of last dose of antibody, and toxicity related to prior antibody therapy must be recovered to Grade ≤ 1.
d. Corticosteroids: If used to modify immune adverse events related to prior therapy, ≥ 14 days must have elapsed since last dose of corticosteroid.
e. Hematopoietic growth factors: ≥ 14 days after the last dose of a long-acting growth factor (e.g. pegfilgrastim) or 7 days for short-acting growth factor. For agents that have known adverse events occurring beyond 7 days after administration, this period must be extended beyond the time during which adverse events are known to occur.
f. Interleukins, Interferons and Cytokines (other than Hematopoietic Growth Factors): >21 days after the completion of interleukins, interferon or cytokines (other than Hematopoietic Growth Factors)
g. Stem cell Infusions (with or without TBI):
   - Allogeneic (non-autologous) bone marrow or stem cell transplant, or any stem cell infusion including DLI or boost infusion:
      o ≥ 60 days after infusion for bone marrow or stem cell transplant and
      o ≥ 4 weeks after infusion for any stem cell infusion including DLI or boost infusion.
      ∘ There must be no evidence of GVHD.
   - Autologous stem cell infusion including boost infusion: ≥ 42 days.
h. Cellular Therapy: ≥ 42 days after the completion of any type of cellular therapy (e.g. modified T cells, NK cells, dendritic cells, etc.)
i. XRT/External Beam Irradiation including Protons: ≥ 14 days after local XRT; ≥ 150 days after TBI, craniospinal XRT or if radiation to ≥ 50% of the pelvis; ≥ 42 days if other substantial BM radiation.
j. Radiopharmaceutical therapy (e.g., radiolabeled antibody, 131I-MIBG): ≥ 42 days after systemically administered radiopharmaceutical therapy.
k. Study specific limitations on prior therapy: small molecule investigational agents: ≥ 14 days and > 5 half-lives must have elapsed from the last dose of the agent, whichever is greater.

8. Organ Function Requirements
a. Adequate Bone Marrow Function Defined as:
- Platelet count ≥ 20,000/mm3 (may receive platelet transfusions). These patients must not be known to be refractory to red cell or platelet transfusion.
- Hemoglobin ≥ 8.0 g/dL at baseline (may receive RBC transfusions).

b. Adequate Renal Function Defined as:
• Creatinine clearance or radioisotope GFR ≥ 70ml/min/1.73 m2 or
• A serum creatinine based on age/gender as follows:

| Age | Maximum Serum Creatinine (mg/dL) | |
|---|---|---|
| | Male | Female |
| 2 to < 6 years | 0.8 | 0.8 |
| 6 to < 10 years | 1 | 1 |
| 10 to < 13 years | 1.2 | 1.2 |
| 13 to < 16 years | 1.5 | 1.4 |
| ≥ 16 years | a. 1.4 | |

The threshold creatinine values in this Table were derived from the Schwartz formula for estimating GFR (Schwartz et al. J. Peds, 106:522, 1985) utilizing child length and stature data published by the CDC.
c. Adequate Liver Function Defined as:
- Bilirubin (sum of conjugated + unconjugated) ≤ 1.5 x upper limit of normal (ULN) for age.
- SGPT (ALT) ≤ 225 U/L. For the purpose of this study, the ULN for SGPT is 45U/L.
- Serum albumin ≥ 2 g/dL.

d. Adequate Cardiac Function Defined As:
- Left ventricular ejection fraction of ≥ 50% by echocardiogram.
- QTc interval by electrocardiogram (EKG) of ≤ 480ms

### Exclusion criteria:

1. Pregnancy or Breast-Feeding
   a. Pregnant or breast-feeding women will not be entered on this study due to risks of fetal and teratogenic adverse events as seen in animal/human studies. Pregnancy tests must be obtained in girls who are post-menarchal. Males or females of reproductive potential may not participate unless they have agreed to use an effective contraceptive method for the duration of study therapy and for 4 months after the last dose of enasidenib. Abstinence is an acceptable method of birth control.
   b. It is not known if enasidenib is present in breast milk. Breastfeeding is not recommended during therapy or for at least 30 days after the last dose of enasidenib.
2. Concomitant Medications:
   a. Corticosteroids: Patients receiving corticosteroids who have not been on a stable or decreasing dose of corticosteroid for at least 7 days prior to enrollment are not eligible. If used to modify immune adverse events related to prior therapy, ≥ 14 days must have elapsed since last dose of corticosteroid. The use of corticosteroids to manage the side effect of IDH-DS, is permitted on study.
   b. Investigational Drugs: Patients who are currently receiving another investigational drug are not eligible.
   c. Anti-cancer Agents: Patients who are currently receiving other anti-cancer agents are not eligible [except leukemia patients receiving hydroxyurea, which may be continued until 24 hours prior to start of protocol therapy; the use of hydroxyurea to manage the side effect of IDH- DS is permitted on study].
   d. Anti-GVHD agents post-transplant.
   e. Patients who are receiving cyclosporine, tacrolimus or other agents to prevent graft-versus-host disease post bone marrow transplant are not eligible for this trial.
3. Currently taking medications that are mainly metabolized by CYP3A4/5, CYP2C8, CYP2C9, CYP2C19, CYP2D6 or the drug transporters Pgp (MDR1), BCRP, OATP1B 1, OATP1B3, OCT1 and OCT2 and have a low therapeutic index that cannot be discontinued at least 7 days or 5 x reported elimination half-life prior to start of treatment with any of the investigational drugs and for the duration of the study.

### ADDITIONAL INCLUSION, AND ENRICHMENT CRITERIA

Patient must have documented IDH2 gene-mutated disease and had at least 2 prior induction therapy

Patient with IDH2 germline mutations and significant clinical deficit of the disease will be allowed

For patients with documented IDH2 mutation, the inclusion criteria of extensive molecular profiling of the recurrent tumor may be waved.

### INVESTIGATIONAL MEDICINAL PRODUCTS (IMPS)

Enasidenib orally administered on a continuous dosing once daily (QD) per 28 day cycle.

### PRIMARY EVALUATION CRITERIA

### Trial End-points

1. The recommended phase II dose (RP2D) will be defined as the adult recommended dose (adjusted for weight or BSA) if toxicity and PK profiling are similar in children and in adults, or a higher dose, providing it is below or equal to the maximum tolerated dose (MTD).
2. The maximum tolerated dose (MTD) will be defined as the dose associated with or closest to 25% of DLTs in cycle 1.
3. Dose Limiting Toxicities (DLT) will be defined using CTCAE v4.03.
4. Overall response rate (ORR); duration of response (DOR) will be defined as the time period between the first documented response (PR or CR) and the time of progression, according to RECIST v1.1, RANO criteria for patients with HGG, INRC criteria for patients with NB, etc.
5. Duration of response for patients free of progression at the cut-off date will be censored at the last imaging response scan date; progression-free survival (PFS) will be defined as the time from treatment initiation until the date of first documented progression or death from any cause. Patients alive and free of progression at the cut-off date will be censored at the last assessment date.
6. Adverse events according to the NCI CTCAE V4.03 in all cycles of treatment.
7. PK parameters, including but not limited to plasma concentration time profiles, AUClast, AUCtau, Cmin, Cmax, Tmax, Clearance, Half-life time.
8. To explore relationship between the molecular profile of the tumor samples, circulating tumor DNA and tumor growth measured as modification of the sum of the diameters of the target lesions over time.

### STUDY DURATION

Patients will continue study treatment until disease progression, unacceptable toxicity, inadequate toxicity-benefit ratio, patient/parents choice.
Planned recruitment period: 108 months
Follow-up period: 18 months after the last inclusion
Planned study duration: 126 months

### Example 11: Proof-of-Concept Therapeutic Stratification Trial of Molecular Anomalies in Relapsed or Refractory Tumors

### OBJECTIVES

### Primary Objectives:

1. To evaluate the safety, antitumor activity, and pharmacokinetic of enasidenib in relapsed or refractory acute myeloid leukaemia paediatric patients with isocitrate dehydrogenase 2 (IDH2) mutation from 2 to leass that 18 years of age.

### Secondary Objectives:

To characterize the PK/pharmacodynamic relationships of AG-221 and 2-hydroxygluturate (2HG).
- Signal of activity based on the 2003 revised International Working Group criteria for AML.

### STUDY DESIGN

This is an international multi-center, single-arm, open-label, prospective, phase II dose-validation study with a RP2D confirmation part, which is open to being expanded to refine an efficacy assessment for each agent.

For all patients, an extensive molecular analysis of their tumor tissue performed prior to study entry will be requested that will serve to select the most appropriate treatment.
If no molecular alteration in the pathways selected for this trial are identified, patients may still be enrolled into this study according to physician discretion, if a strong scientific rationale exists to support the notion that the patient may derive clinical benefit, and if the patient fulfills all other inclusion and no exclusion criteria.

### STUDY POPULATION

9. Age: Patients must be ≥ 24 months and ≤ 18 years of age at the time of study enrollment.

10. Diagnosis: Patients with AML in the 2^{nd} or greater relapse or with refractory AML after ≥ two prior induction attempts.
a. Each block of chemotherapy (i.e. ADE, MA) is a separate re- induction attempt.
b. Donor lymphocyte infusion (DLI) is considered a re-induction attempt.
c. AML associated with Down Syndrome or t(15;17) are not eligible for study

11. Performance Level: Karnofsky ≥ 50% for patients > 16 years of age and Lansky ≥ 50 for patients ≤ 16 years of age.

12. Disease Status: Bone marrow assessment must have at least one of the following:
a. ≥ 5 % blasts by morphology and/or flow cytometry
b. Minimal residual disease by flow cytometry
c. Any molecular evidence of disease
   Patient must have documented IDH2 gene-mutated disease obtained from a peripheral blood or bone marrow sample at the time of diagnosis and/or relapsed/refractory disease. Mutations in IDH2 at R140 or R172 are eligible for enrollment.

13. Therapeutic Options: Patient's current disease state must be one for which there is no known curative therapy or therapy proven to prolong survival with an acceptable quality of life.

14. CNS Status: No intrathecal therapy will be permitted on study. Subjects with known CNS leukemia or has clinical symptoms suggesting active central nervous system (CNS) leukemia will not be eligible Therefore, only patients with CNS1 disease will be eligible for study. CNS1 status is defined as absence of blasts in cerebral spinal fluid (CSF) on cytospin preparation, regardless of the number of WBCs in the CSF.

15. Prior Therapy: Patients must have fully recovered from the acute toxic effects of all prior anti-cancer therapy to ≤ Grade 2 prior to enrollment, and must meet the following minimum duration from prior anti-cancer directed therapy prior to enrollment. If after the required timeframe, the numerical eligibility criteria are met, e.g., blood count criteria, the patient is considered to have recovered adequately.
a. Cytotoxic chemotherapy or other anti-cancer agents known to be myelosuppressive.
   - ≥ 14 days must have elapsed after the completion of other cytotoxic therapy, with the exception of hydroxyurea. Additionally, patients must have fully recovered from all acute toxic effects of prior therapy.
   - Intrathecal chemotherapy must be completed ≥ 72 hours prior to the start of the first cycle of treatment.
   NOTE: Cytoreduction with hydroxyurea must be discontinued ≥ 24 hours prior to the start of protocol therapy.
b. Anti-cancer agents not known to be myelosuppressive (e.g. not associated with reduced platelet or ANC counts): ≥ 7 days after the last dose of agent.
c. Antibodies: ≥ 21 days must have elapsed from infusion of last dose of antibody, and toxicity related to prior antibody therapy must be recovered to Grade ≤ 1.
d. Corticosteroids: If used to modify immune adverse events related to prior therapy, ≥ 14 days must have elapsed since last dose of corticosteroid.
e. Hematopoietic growth factors: ≥ 14 days after the last dose of a long-acting growth factor (e.g. pegfilgrastim) or 7 days for short-acting growth factor. For agents that have known adverse events occurring beyond 7 days after administration, this period must be extended beyond the time during which adverse events are known to occur.
f. Interleukins, Interferons and Cytokines (other than Hematopoietic Growth Factors): ≥21 days after the completion of interleukins, interferon or cytokines (other than Hematopoietic Growth Factors)
g. Stem cell Infusions (with or without TBI):
   - Allogeneic (non-autologous) bone marrow or stem cell transplant, or any stem cell infusion including DLI or boost infusion:
      o ≥ 60 days after infusion for bone marrow or stem cell transplant and
      o ≥ 4 weeks after infusion for any stem cell infusion including DLI or boost infusion.
      ∘ There must be no evidence of GVHD.
   - Autologous stem cell infusion including boost infusion: ≥ 42 days.
h. Cellular Therapy: ≥ 42 days after the completion of any type of cellular therapy (e.g. modified T cells, NK cells, dendritic cells, etc.)
i. XRT/External Beam Irradiation including Protons: ≥ 14 days after local XRT; ≥ 150 days after TBI, craniospinal XRT or if radiation to ≥ 50% of the pelvis; ≥ 42 days if other substantial BM radiation.
j. Radiopharmaceutical therapy (e.g., radiolabeled antibody, 131I-MIBG): ≥ 42 days after systemically administered radiopharmaceutical therapy.
k. Study specific limitations on prior therapy: small molecule investigational agents: ≥ 14 days and > 5 half-lives must have elapsed from the last dose of the agent, whichever is greater.

16. Organ Function Requirements
a. Adequate Bone Marrow Function Defined as:
- Platelet count ≥ 20,000/mm3 (may receive platelet transfusions). These patients must not be known to be refractory to red cell or platelet transfusion.
- Hemoglobin ≥ 8.0 g/dL at baseline (may receive RBC transfusions).

b. Adequate Renal Function Defined as:
• Creatinine clearance or radioisotope GFR ≥ 70ml/min/1.73 m2 or
• A serum creatinine based on age/gender as follows:

| Age | Maximum Serum Creatinine (mg/dL) | |
|---|---|---|
| | Male | Female |
| 2 to < 6 years | 0.8 | 0.8 |
| 6 to < 10 years | 1 | 1 |
| 10 to < 13 years | 1.2 | 1.2 |
| 13 to < 16 years | 1.5 | 1.4 |
| ≥ 16 years | b. 1.4 | |

The threshold creatinine values in this Table were derived from the Schwartz formula for estimating GFR (Schwartz et al. J. Peds, 106:522, 1985) utilizing child length and stature data published by the CDC.
c. Adequate Liver Function Defined as:
- Bilirubin (sum of conjugated + unconjugated) ≤ 1.5 x upper limit of normal (ULN) for age.
- SGPT (ALT) ≤ 225 U/L. For the purpose of this study, the ULN for SGPT is 45U/L.
- Serum albumin ≥ 2 g/dL.

d. Adequate Cardiac Function Defined As:
- Left ventricular ejection fraction of ≥ 50% by echocardiogram.
- QTc interval by electrocardiogram (EKG) of ≤ 480ms.

### Exclusion criteria:

1. Pregnancy or Breast-Feeding
   a. Pregnant or breast-feeding women will not be entered on this study due to risks of fetal and teratogenic adverse events as seen in animal/human studies. Pregnancy tests must be obtained in girls who are post-menarchal. Males or females of reproductive potential may not participate unless they have agreed to use an effective contraceptive method for the duration of study therapy and for 4 months after the last dose of enasidenib. Abstinence is an acceptable method of birth control.
   b. It is not known if enasidenib is present in breast milk. Breastfeeding is not recommended during therapy or for at least 30 days after the last dose of enasidenib.
2. Concomitant Medications:
   a. Corticosteroids: Patients receiving corticosteroids who have not been on a stable or decreasing dose of corticosteroid for at least 7 days prior to enrollment are not eligible. If used to modify immune adverse events related to prior therapy, ≥ 14 days must have elapsed since last dose of corticosteroid. The use of corticosteroids to manage the side effect of IDH-DS, is permitted on study.
   b. Investigational Drugs: Patients who are currently receiving another investigational drug are not eligible.
   c. Anti-cancer Agents: Patients who are currently receiving other anti-cancer agents are not eligible [except leukemia patients receiving hydroxyurea, which may be continued until 24 hours prior to start of protocol therapy; the use of hydroxyurea to manage the side effect of IDH- DS is permitted on study].
   d. Anti-GVHD agents post-transplant.
   e. Patients who are receiving cyclosporine, tacrolimus or other agents to prevent graft-versus-host disease post bone marrow transplant are not eligible for this trial.
3. Currently taking medications that are mainly metabolized by CYP3A4/5, CYP2C8, CYP2C9, CYP2C19, CYP2D6 or the drug transporters Pgp (MDR1), BCRP, OATP1B 1, OATP1B3, OCT1 and OCT2 and have a low therapeutic index that cannot be discontinued at least 7 days or 5 x reported elimination half-life prior to start of treatment with any of the investigational drugs and for the duration of the study.

### ADDITIONAL INCLUSION, AND ENRICHMENT CRITERIA

Patient must have documented IDH2 gene-mutated disease and had at least 2 prior induction therapy

Patient with IDH2 germline mutations and significant clinical deficit of the disease will be allowed

For patients with documented IDH2 mutation, the inclusion criteria of extensive molecular profiling of the recurrent tumor may be waved.

### INVESTIGATIONAL MEDICINAL PRODUCTS (IMPS)

Enasidenib is administered orally on a continuous dosing once daily (QD) per 28 day cycle.

### FORMULATIONS/ROUTES OF ADMINISTRATION

A sprinkle formulation provided herein is administered orally with soft food to deliver a dose of 20 mg, 40 mg, 60 mg, 80 mg or 100 mg Compound 1C.

### PRIMARY EVALUATION CRITERIA

### Trial End-points

1. The recommended phase II dose (RP2D) will be defined as the adult recommended dose (adjusted for weight or BSA) if toxicity and PK profiling are similar in children and in adults, or a higher dose, providing it is below or equal to the maximum tolerated dose (MTD).
2. The maximum tolerate dose (MTD) will be defined as the dose associated with or closest to 25% of DLTs in cycle 1.
3. Dose Limiting Toxicities (DLT) will be defined using CTCAE v4.03.
4. Overall response rate (ORR); duration of response (DOR) will be defined as the time period between the first documented response (PR or CR) and the time of progression, according to RECIST v1.1, RANO criteria for patients with HGG, INRC criteria for patients with NB, etc.
5. Duration of response for patients free of progression at the cut-off date will be censored at the last imaging response scan date; progression-free survival (PFS) will be defined as the time from treatment initiation until the date of first documented progression or death from any cause. Patients alive and free of progression at the cut-off date will be censored at the last assessment date.
6. Adverse events according to the NCI CTCAE V4.03 in all cycles of treatment.
7. PK parameters, including but not limited to plasma concentration time profiles, AUClast, AUCtau, Cmin, Cmax, Tmax, Clearance, Half-life time.
8. To explore relationship between the molecular profile of the tumor samples, circulating tumor DNA and tumor growth measured as modification of the sum of the diameters of the target lesions over time.

### STUDY DURATION

Patients will continue study treatment until disease progression, unacceptable toxicity, inadequate toxicity-benefit ratio, patient/parents choice.
Planned recruitment period: 108 months
Follow-up period: 18 months after the last inclusion
Planned study duration: 126 months

The examples set forth above are provided to give those of ordinary skill in the art with a complete disclosure and description of how to make and use the claimed embodiments, and are not intended to limit the scope of what is disclosed herein.

## Claims

1. A minitablet comprising about 9% to about 10% 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate or a solid form thereof (Compound 1A), about 42% to about 45% microcrystalline cellulose, about 26.5% to about 28.5% mannitol, about 1% to about 3% hydroxypropyl cellulose, about 6% to about 10% sodium starch glycolate, about 0.5% to about 1.5% sodium lauryl sulfate, about 1% to about 3% colloidal silicon dioxide, about 0.5% to about 2% hypromellose acetate succinate, about 3.5% to 5% sucralose, and about 1% to about 3% magnesium stearate, where the percentages are by weight based on total weight of the minitablet.

2. The minitablet of claim 1 comprising about 9.62% Compound 1A, about 43.24% microcrystalline cellulose, about 27.24% mannitol, about 2% hydroxypropyl cellulose, about 8% sodium starch glycolate, about 1% sodium lauryl sulfate, about 2% colloidal silicon dioxide, about 1% hypromellose acetate succinate, about 4.4% sucralose, and about 1.5% magnesium stearate, where the percentages are by weight based on total weight of the minitablet.

3. The minitablet of claim 1 or 2
(a) having a weight of about 1.67 mg; and/or
(b) having a diameter of about 1.2 mm; and/or
(c) having a height of about 1.2 mm.

4. The minitablet of any one of claims 1 to 3 having a solid fraction of about 0.9 to 0.95 and a tensile strength of 1.0 to 1.6 MPa.

5. The minitablet of any one of claims 1 to 4 comprising about 9.62% solid form 3 of 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate.

6. A capsule comprising a minitablet of any one of claims 1 to 5.

7. The capsule of claim 6 comprising about 100-500 minitablets, optionally comprising about 150, 300 or 450 minitablets.

8. A process for preparing a minitablet comprising i) blending intragranular components to obtain an intragranular blend, ii) roller compacting the intragranular blend to obtain roller compacted granules, iii) blending extragranular components to obtain an extragranular blend, iv) blending the roller compacted granules with the extragranular blend to obtain a final blend, and v) compressing the final blend to obtain the minitablet, wherein the intragranular blend comprises 2-methyl-1-[(4-[6-(trifluoromethyl)pyridin-2-yl]-6-{[2-(trifluoromethyl)pyridin-4-yl]amino}-1,3,5-triazin-2-yl)amino]propan-2-ol methanesulfonate (Compound 1A), a binder, a sweet diluent, a disintegrant, a wetting agent, a flow agent, a stabilizer, a high intensity sweetener and a lubricant, and the extragranular blend comprises a binder, a disintegrant, and a flow agent.

9. The process of claim 8, comprising: i) blending Compound 1A with intragranular components including, microcrystalline cellulose, hydroxypropyl cellulose, sodium starch glycolate, sodium lauryl sulfate, colloidal SiO₂, mannitol, sucralose, hypromellose acetate succinate to obtain an intragranular blend; ii) blending the intragranular blend with magnesium stearate to obtain a lubricated intragranular blend; iii) roller compacting the lubricated intragranular blend to obtain roller compacted granules; iv) blending the roller compacted granules with an extragranular blend to obtain a final blend, wherein the extragranular blend is obtained by blending microcrystalline cellulose, sodium starch glycolate and colloidal SiO₂; and v) compressing the final blend to obtain the minitablet.

10. A process of preparing a capsule comprising filling the minitablets prepared by the process of claim 8 or 9 into a capsule.

11. The process of any one of claims 8 to 10, wherein the intragranular blend comprises solid form 3 of 2-methyl-1-[(4-[6-(trifluoromethyl)pyridin-2-yl]-6- {[2-(trifluoromethyl)pyridin-4-yl]amino}-1,3,5-triazin-2-yl)amino]propan-2-ol methanesulfonate.

12. A minitablet of any one of claims 1 to 5 or a capsule of claim 6 or 7 for use in a method of treating a disease selected from a hematologic malignancy and a solid tumor, each **characterized by** the presence of a mutant allele of IDH2, wherein the method comprises administering said minitablet or said capsule to a pediatric patient having the disease, optionally
(a) wherein the disease is **characterized by** the presence of a mutant allele of IDH2 and the absence of a mutant allele of FLT3; or
(b) wherein the disease is **characterized by** the presence of a mutant allele of IDH2 and the absence of a mutant allele of NRAS.

13. The minitablet or the capsule for use of claim 12,
(a) wherein the disease is a hematologic malignancy; and/or
(b) wherein the hematologic malignancy is selected from acute myelogenous leukemia, myelodysplastic syndrome, chronic myelomonocytic leukemia myeloid sarcoma, multiple myeloma, lymphoma, angioimmunoblastic T-cell lymphoma, blastic plasmacytoid dendritic cell neoplasm and myeloproliferative neoplasm, each **characterized by** the presence of a mutant allele of IDH2; and/or
(c) wherein the hematologic malignancy is acute myelogenous leukemia.

14. The minitablet or the capsule for use of claim 12,
(a) wherein the disease is myelodysplastic syndrome; or
(b) wherein the disease is **characterized by** the presence of a mutant allele of IDH2 and a mutant allele of at least one second gene, wherein the second gene is selected from the group consisting of ASXL1 and SRSF2; or
(c) wherein the disease is **characterized by** the presence of a mutant allele of IDH2 and the absence of a mutant allele of at least one other gene, wherein the other gene is selected from the group consisting of KRAS, TP53, SETBP1, U2AF1, TCF3, STAG2, NRAS, JAK2 and BRAF.

15. The minitablet or the capsule for use of claim 12, wherein the solid tumor is selected from glioma, melanoma, chondrosarcoma, and cholangiocarcinoma, each **characterized by** the presence of a mutant allele of IDH2.

16. The minitablet or the capsule for use of any one of claims 12 to 14(b), wherein the disease is relapsed or refractory.

17. The minitablet or the capsule for use of any one of claims 12 to 16, wherein the method further comprises administering a second active agent.

## Patentansprüche

1. Minitablette, umfassend etwa 9 % bis etwa 10 % 2-Methyl-1-[(4-[6-(trifluormethyl)pyridin-2-yl]-6-{[2-(trifluormethyl)pyridin-4-yl]amino}-1,3,5-triazin-2-yl)amino]propan-2-olmethansulfonat oder eine feste Form davon (Verbindung 1A), etwa 42 % bis etwa 45 % mikrokristalline Cellulose, etwa 26,5 % bis etwa 28,5 % Mannit, etwa 1 % bis etwa 3 % Hydroxypropylcellulose, etwa 6 % bis etwa 10 % Natriumstärkeglycolat, etwa 0,5 % bis etwa 1,5 % Natriumlaurylsulfat, etwa 1 % bis etwa 3 % kolloidales Siliciumdioxid, etwa 0,5 % bis etwa 2 % Hypromelloseacetatsuccinat, etwa 3,5 % bis etwa 5 % Sucralose und etwa 1 % bis etwa 3 % Magnesiumstearat, wobei die Prozentzahlen Gewichtsprozent bezogen auf das Gesamtgewicht der Minitablette bedeuten.

2. Minitablette nach Anspruch 1, umfassend etwa 9,62 % Verbindung 1A, etwa 43,24 % mikrokristalline Cellulose, etwa 27,24 % Mannit, etwa 2 % Hydroxypropylcellulose, etwa 8 % Natriumstärkeglycolat, etwa 1 % Natriumlaurylsulfat, etwa 2 % kolloidales Siliciumdioxid, etwa 1 % Hypromelloseacetatsuccinat, etwa 4,4 % Sucralose und etwa 1,5 % Magnesiumstearat, wobei die Prozentzahlen Gewichtsprozent bezogen auf das Gesamtgewicht der Minitablette bedeuten.

3. Minitablette nach Anspruch 1 oder 2,
(a) aufweisend ein Gewicht von etwa 1,67 mg; und/oder
(b) aufweisend einen Durchmesser von etwa 1,2 mm; und/oder
(c) aufweisend eine Höhe von etwa 1,2 mm.

4. Minitablette nach einem der Ansprüche 1 bis 3, aufweisend eine Feststofffraktion von etwa 0,9 bis 0,95 und eine Festigkeit von 1,0 bis 1,6 MPa.

5. Minitablette nach einem der Ansprüche 1 bis 4, umfassend etwa 9,62 % feste Form 3 von 2-Methyl-1-[(4-[6-(trifluormethyl)pyridin-2-yl]-6-{[2-(trifluormethyl)pyridin-4-yl]amino}-1,3,5-triazin-2-yl)amino]propan-2-olmethansulfonat.

6. Kapsel, umfassend eine Minitablette nach einem der Ansprüche 1 bis 5.

7. Kapsel nach Anspruch 6, umfassend etwa 100-500 Minitabletten, gegebenenfalls umfassend etwa 150, 300 oder 450 Minitabletten.

8. Verfahren zum Herstellen einer Minitablette, umfassend i) Mischen intragranulärerKomponenten unter Erhalt einer intragranulären Mischung, ii) Walzenkompaktieren der intragranulären Mischung unter Erhalt von walzenkompaktiertem Granulat, iii) Mischen extragranulärer Komponenten unter Erhalt einer extragranulären Mischung, iv) Mischen des walzenkompaktierten Granulats mit der extragranulären Mischung unter Erhalt einer letzten Mischung und v) Verpressen der letzten Mischung unter Erhalt der Minitablette, wobei die intragranuläre Mischung 2-Methyl-1-[(4-[6-(trifluormethyl)pyridin-2-yl]-6-{[2-(trifluormethyl)pyridin-4-yl]amino}-1,3,5-triazin-2-yl)amino]propan-2-olmethansulfonat (Verbindung 1A), ein Bindemittel, ein süßes Verdünnungsmittel, ein Sprengmittel, ein Netzmittel, ein Fließmittel, einen Stabilisator, einen hochintensiven Süßstoff und ein Schmiermittel umfasst und die extragranuläre Mischung ein Bindemittel, ein Sprengmittel und ein Fließmittel umfasst.

9. Verfahren nach Anspruch 8, umfassend: i) Mischen von Verbindung 1A mit intragranulären Komponenten, einschließlich mikrokristalliner Cellulose, Hydroxypropylcellulose, Natriumstärkeglycolat, Natriumlaurylsulfat, kolloidalem SiO₂, Mannit, Sucralose, Hypromelloseacetatsuccinat, unter Erhalt einer intragranulären Mischung; ii) Mischen der intragranulären Mischung mit Magnesiumstearat unter Erhalt einer geschmierten intragranulären Mischung; iii) Walzenkompaktieren der geschmierten intragranulären Mischung unter Erhalt von walzenkompaktiertem Granulat; iv) Mischen des walzenkompaktierten Granulats mit einer extragranulären Mischung unter Erhalt einer letzten Mischung, wobei die extragranuläre Mischung durch Mischen von mikrokristalliner Cellulose, Natriumstärkeglycolat und kolloidalem SiO₂ erhalten wird; und v) Verpressen der letzten Mischung unter Erhalt der Minitablette.

10. Verfahren zum Herstellen einer Kapsel, umfassend Füllen der mit dem Verfahren nach Anspruch 8 oder 9 hergestellten Minitabletten in eine Kapsel.

11. Verfahren nach einem der Ansprüche 8 bis 10, wobei die intragranuläre Mischung feste Form 3 von 2-Methyl-1-[(4-[6-(trifluormethyl)pyridin-2-yl]-6-{[2-(trifluormethyl)pyridin-4-yl]amino}-1,3,5-triazin-2-yl)amino]propan-2-olmethansulfonat umfasst.

12. Minitablette nach einem der Ansprüche 1 bis 5 oder Kapsel nach Anspruch 6 oder 7 zur Verwendung bei einer Methode zum Behandeln einer Krankheit, die aus einem hämatologischem Malignom und einem soliden Tumor, jeweils **gekennzeichnet durch** das Vorhandensein eines mutierten IDH2-Allels, ausgewählt ist, wobei die Methode Verabreichen der Minitablette oder der Kapsel an Kinder mit der Krankheit umfasst, gegebenenfalls
(a) wobei die Krankheit **durch** das Vorhandensein eines mutierten IDH2-Allels und die Abwesenheit eines mutierten FLT3-Allels gekennzeichnet ist; oder
(b) wobei die Krankheit **durch** das Vorhandensein eines mutierten IDH2-Allels und die Abwesenheit eines mutierten NRAS-Allels gekennzeichnet ist.

13. Minitablette oder Kapsel zur Verwendung nach Anspruch 12,
(a) wobei es sich bei der Krankheit um ein hämatologisches Malignom handelt; und/oder
(b) wobei das hämatologische Malignom aus akuter myeloischer Leukämie, myelodysplastischem Syndrom, chronischer Myelomonozytenleukämie, Myelosarkom, multiplem Myelom, Lymphom, angioimmunoblastischem T-Zell-Lymphom, blastischer plasmazytoider Neoplasie dendritischer Zellen und myeloproliferativer Neoplasie, jeweils **gekennzeichnet durch** das Vorhandensein eines mutierten IDH2-Allels, ausgewählt ist; und/oder
(c) wobei es sich bei dem hämatologischen Malignom um akute myeloische Leukämie handelt.

14. Minitablette oder Kapsel zur Verwendung nach Anspruch 12,
(a) wobei es sich bei der Krankheit um myelodysplastisches Syndrom handelt; oder
(b) wobei die Krankheit durch das Vorhandensein eines mutierten IDH2-Allels und mindestens eines zweiten Gens mit einem mutierten Allel gekennzeichnet ist, wobei das zweite Gen aus der Gruppe bestehend aus ASXL1 und SRSF2 ausgewählt ist; oder
(c) wobei die Krankheit durch das Vorhandensein eines mutierten IDH2-Allels und die Abwesenheit mindestens eines anderen Gens mit einem mutierten Allel gekennzeichnet ist, wobei das andere Gen aus der Gruppe bestehend aus KRAS, TP53, SETBP1, U2AF1, TCF3, STAG2, NRAS, JAK2 und BRAF ausgewählt ist.

15. Minitablette oder Kapsel zur Verwendung nach Anspruch 12, wobei der solide Tumor aus Gliom, Melanom, Chondrosarkom und Cholangiokarzinom, jeweils **gekennzeichnet durch** das Vorhandensein eines mutierten IDH2-Allels, ausgewählt ist.

16. Minitablette oder Kapsel zur Verwendung nach einem der Ansprüche 12 bis 14(b), wobei die Krankheit rezidiviert oder refraktär ist.

17. Minitablette oder Kapsel zur Verwendung nach einem der Ansprüche 12 bis 16, wobei das Verfahren ferner Verabreichen eines zweiten Wirkstoffs umfasst.

## Revendications

1. Mini-comprimé comprenant environ 9 % à environ 10 % de méthanesulfonate de 2-méthyl-1-[(4-[6-(trifluorométhyl)pyridin-2-yl]-6-{[2-(trifluorométhyl)pyridin-4-yl]amino}-1,3,5-triazin-2-yl)amino]propan-2-ol ou d'une forme solide de celui-ci (composé 1A), environ 42 % à environ 45 % de cellulose microcristalline, environ 26,5 % à environ 28,5 % de mannitol, environ 1 % à environ 3 % d'hydroxypropylcellulose, environ 6 % à environ 10 % de glycolate d'amidon sodique, environ 0,5 % à environ 1,5 % de laurylsulfate de sodium, environ 1 % à environ 3 % de dioxyde de silicium colloïdal, environ 0,5 % à environ 2 % d'acétate succinate d'hypromellose, environ 3,5 % à 5 % de sucralose et environ 1 % à environ 3 % de stéarate de magnésium, les pourcentages étant en poids sur la base du poids total du mini-comprimé.

2. Mini-comprimé selon la revendication 1, comprenant environ 9,62 % de composé 1A, environ 43,24 % de cellulose microcristalline, environ 27,24 % de mannitol, environ 2 % d'hydroxypropylcellulose, environ 8 % de glycolate d'amidon sodique, environ 1 % de laurylsulfate de sodium, environ 2 % de dioxyde de silicium colloïdal, environ 1 % de succinate d'acétate d'hypromellose, environ 4,4 % de sucralose et environ 1,5 % de stéarate de magnésium, les pourcentages étant en poids sur la base du poids total du mini-comprimé.

3. Mini-comprimé selon la revendication 1 ou 2
(a) ayant un poids d'environ 1,67 mg ; et/ou
(b) ayant un diamètre d'environ 1,2 mm ; et/ou
(c) ayant une hauteur d'environ 1,2 mm.

4. Mini-comprimé selon l'une quelconque des revendications 1 à 3, ayant une fraction solide d'environ 0,9 à 0,95 et une résistance à la traction de 1,0 à 1,6 MPa.

5. Mini-comprimé selon l'une quelconque des revendications 1 à 4, comprenant environ 9,62 % de forme solide 3 du méthanesulfonate de 2-méthyl-1-[(4-[6-(trifluorométhyl)pyridin-2-yl]-6-{[2-(trifluorométhyl)pyridin-4-yl]amino}-1,3,5-triazin-2-yl)amino]propan-2-ol.

6. Capsule comprenant un mini-comprimé selon l'une quelconque des revendications 1 à 5.

7. Capsule selon la revendication 6, comprenant environ 100-500 mini-comprimés, comprenant éventuellement environ 150, 300 ou 450 mini-comprimés.

8. Procédé de préparation d'un mini-comprimé comprenant i) le mélange de composants intragranulaires pour obtenir un mélange intragranulaire, ii) le compactage au rouleau du mélange intragranulaire pour obtenir des granules compactés au rouleau, iii) le mélange de composants extragranulaires pour obtenir un mélange extragranulaire, iv) le mélange des granules compactés au rouleau avec le mélange extragranulaire pour obtenir un mélange final, et v) la compression du mélange final pour obtenir le mini-comprimé, le mélange intragranulaire comprenant du méthanesulfonate de 2-méthyl-1-[(4-[6-(trifluorométhyl)pyridin-2-yl]-6-{ [2-(trifluorométhyl)pyridin-4-yl]amino}-1,3,5-triazin-2-yl)amino]propan-2-ol (composé 1A), un liant, un diluant sucré, un désintégrant, un agent mouillant, un agent d'écoulement, un stabilisant, un édulcorant à haute intensité et un lubrifiant, et le mélange extragranulaire comprenant un liant, un désintégrant et un agent d'écoulement.

9. Procédé selon la revendication 8, comprenant : i) le mélange du composé 1A avec des composants intragranulaires comprenant de la cellulose microcristalline, de l'hydroxypropylcellulose, du glycolate d'amidon sodique, du laurylsulfate de sodium, du SiO₂ colloïdal, du mannitol, du sucralose, de l'acétate succinate d'hypromellose pour obtenir un mélange intragranulaire ; ii) le mélange du mélange intragranulaire avec du stéarate de magnésium pour obtenir un mélange intragranulaire lubrifié ; iii) le compactage au rouleau du mélange intragranulaire lubrifié pour obtenir des granules compactés au rouleau ; iv) le mélange des granules compactés au rouleau avec un mélange extragranulaire pour obtenir un mélange final, dans lequel le mélange extragranulaire est obtenu par mélange de cellulose microcristalline, du glycolate d'amidon sodique et de SiO₂ colloïdal ; et v) la compression du mélange final pour obtenir le mini-comprimé.

10. Procédé de préparation d'une capsule comprenant le remplissage des mini-comprimés préparés par le procédé selon la revendication 8 ou 9 dans une capsule.

11. Procédé selon l'une quelconque des revendications 8 à 10, dans lequel le mélange intragranulaire comprend la forme solide 3 du méthanesulfonate de 2-méthyl-1-[(4-[6-(trifluorométhyl)pyridin-2-y1]-6-{[2-(trifluorométhyl)pyridin-4-yl]amino}-1,3,5-triazin-2-yl)amino]propan-2-ol.

12. Mini-comprimé selon l'une quelconque des revendications 1 à 5 ou capsule selon la revendication 6 ou 7 pour utilisation dans un procédé de traitement d'une maladie choisie parmi une malignité hématologique et une tumeur solide, caractérisées chacune par la présence d'un allèle mutant de IDH2, dans lequel le procédé comprend l'administration dudit mini-comprimé ou de ladite capsule à un patient pédiatrique souffrant de la maladie, éventuellement
(a) dans lequel la maladie est **caractérisée par** la présence d'un allèle mutant de IDH2 et l'absence d'un allèle mutant de FLT3 ; ou
(b) dans lequel la maladie est **caractérisée par** la présence d'un allèle mutant de IDH2 et l'absence d'un allèle mutant de NRAS.

13. Mini-comprimé ou capsule pour utilisation selon la revendication 12,
(a) où la maladie est une malignité hématologique ; et/ou
(b) où la malignité hématologique est choisie parmi la leucémie myélogène aiguë, le syndrome myélodysplasique, la leucémie myélomonocytaire chronique, le sarcome myéloïde, le myélome multiple, le lymphome, le lymphome T angio-immunoblastique, le néoplasme blastique des cellules dendritiques plasmocytoïdes et le néoplasme myéloprolifératif, chacun **caractérisé par** la présence d'un allèle mutant de IDH2 ; et/ou
(c) où la malignité hématologique est une leucémie myélogène aiguë.

14. Mini-comprimé ou capsule pour utilisation selon la revendication 12,
(a) où la maladie est le syndrome myélodysplasique ; ou
(b) où la maladie est **caractérisée par** la présence d'un allèle mutant de IDH2 et d'un allèle mutant d'au moins un second gène, où le second gène est choisi dans le groupe constitué par ASXL1 et SRSF2 ; ou
(c) où la maladie est **caractérisée par** la présence d'un allèle mutant de IDH2 et l'absence d'un allèle mutant d'au moins un autre gène, où l'autre gène est choisi dans le groupe constitué par KRAS, TP53, SETBP1, U2AF1, TCF3, STAG2, NRAS, JAK2 et BRAF.

15. Mini-comprimé ou capsule pour utilisation selon la revendication 12, où la tumeur solide est choisie parmi le gliome, le mélanome, le chondrosarcome et le cholangiocarcinome, caractérisés chacun par la présence d'un allèle mutant de IDH2.

16. Mini-comprimé ou capsule pour utilisation selon l'une quelconque des revendications 12 à 14(b), où la maladie est récidivante ou réfractaire.

17. Mini-comprimé ou capsule pour utilisation selon l'une quelconque des revendications 12 à 16, où le procédé comprend en outre l'administration d'un second agent actif.
